(19) Europäisches Patentamt
European Patent Office
Office européen des brevets

(11) EP 1 455 790 B1

(12) EUROPÄISCHE PATENTSCHRIFT

(45) Veröffentlichungstag und Bekanntmachung des Hinweises auf die Patenterteilung:
**26.03.2008 Patentblatt 2008/13**

(21) Anmeldenummer: **02792799.5**

(22) Anmeldetag: **25.11.2002**

(51) Int Cl.:
*A61K 31/505* (2006.01) *A61K 31/506* (2006.01)
*A61K 31/44* (2006.01) *A61K 31/443* (2006.01)
*A61K 31/4433* (2006.01) *A61P 19/00* (2006.01)
*C07D 239/28* (2006.01) *C07D 213/81* (2006.01)
*C07D 405/14* (2006.01) *C07D 405/12* (2006.01)

(86) Internationale Anmeldenummer:
**PCT/EP2002/013240**

(87) Internationale Veröffentlichungsnummer:
**WO 2003/049738 (19.06.2003 Gazette 2003/25)**

# (54) VERWENDUNG VON PYRIDIN-2,4-DICARBONS UREDIAMIDEN UND PYRIMID IN-4,6-DICARBONS UREDIAMIDEN ZUR SELEKTIVEN INHIBIERUNG VON KOLLAGENASEN

USE OF PYRIDINE-2,4-DICARBOXYLIC ACID DIAMIDES AND OF PYRIMIDINE-4,6-DICARBOXYLIC ACID DIAMIDES FOR SELECTIVE COLLAGENASE INHIBITION

UTILISATION DE DIAMIDES D'ACIDE PYRIDINE-2,4-DICARBOXYLIQUE ET DE DIAMIDES D'ACIDE PYRIMIDINE-4,6-DICARBOXYLIQUE POUR L'INHIBITION SELECTIVE DE COLLAGENASES

(84) Benannte Vertragsstaaten:
**AT BE BG CH CY CZ DE DK EE ES FI FR GB GR IE IT LI LU MC NL PT SE SK TR**

(30) Priorität: **08.12.2001 DE 10160357**

(43) Veröffentlichungstag der Anmeldung:
**15.09.2004 Patentblatt 2004/38**

(73) Patentinhaber: **Sanofi-Aventis Deutschland GmbH**
**65929 Frankfurt am Main (DE)**

(72) Erfinder:
- **WEITHMANN, Klaus-Ulrich**
  **65719 Hofheim (DE)**
- **HABERMANN, Jörg**
  **00040 Ariccia/Roma (IT)**
- **KOGLER, Herbert**
  **61479 Glashütten (DE)**
- **KIRSCH, Reinhard**
  **38100 Braunschweig (DE)**
- **WEHNER, Volkmar**
  **97657 Sandberg (DE)**

(56) Entgegenhaltungen:
**EP-A- 0 418 797** **EP-A- 0 463 592**
**WO-A-02/064568** **WO-A-02/064571**

EP 1 455 790 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft die Verwendung von Pyridin-2,4-dicarbonsäurediamiden und Pyrimidin-4,6-dicarbonsäurediamiden zur selektiven Inhibition der Kollagenase (MMP 13). Die Pyridin-2,4-dicarbonsäurediamide und Pyrimidin-4,6-dicarbonsäurediamide können daher zur Behandlung degenerativer Gelenkerkrankungen eingesetzt werden.

Es ist bekannt, dass Pyrimidin-4,6-dicarbonsäurediamide und 2,4-substituierte Pyridin-N-oxide die Enzyme Prolin- und Lysinhydroxylase inhibieren und damit eine Hemmung der Kollagenbiosynthese durch Beeinflussung der kollagenspezifischen Hydroxylierungsreaktion bewirken (EP 0418797; EP 0463592). Durch diese Hemmung der Kollagenbiosynthese wird ein nicht funktionsfähiges, unter-hydroxyliertes Kollagenmolekül gebildet, das von den Zellen nur in geringer Menge in den extrazellularen Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.
In Erkrankungen wie Osteoarthritis und Rheuma findet eine Zerstörung des Gelenkes statt, besonders bedingt durch den proteolytischen Abbau von Kollagen durch Kollagenasen. Kollagenasen gehören zur Superfamilie der Metalloproteinasen (MP) bzw. Matrix-Metallproteinasen (MMP). MMP's spalten Kollagen, Laminin, Proteoglykane, Elastin oder Gelatin unter physiologischen Bedingungen und spielen daher eine wichtige Rolle im Knochen und Bindegewebe. Eine Vielzahl von verschiedenen Inhibitoren der MMP's, bzw. der Kollagenasen sind bekannt EP 0 606 046; WO94/28889). Nachteile der bekannten Inhibitoren der MMP's sind häufig die mangelnde Spezifität der Hemmung für nur eine Klasse der MMP's. Daher hemmen die meisten MMP-Inhibitoren mehrere MMP's gleichzeitig, weil die katalytische Domäne der MMP's eine ähnliche Struktur aufweist. Demzufolge wirken die Inhibitoren in unerwünschter Weise auf viele Enzyme, auch solche mit vitaler Funktion ein (Massova I., et al., The FASEB Journal (1998) 12, 1075-1095). Aus den Patentanmeldungen WO02/064568 und WO02/064571, die unter Art. 54 (3) Europäisches Patentübereinkommen fallen, ist bekannt, dass bestimmte Pyridin-2,4-dicarbonsäurediamide und Pyrimidin-4,6-dicarbonsäurediamide allosterische Inhibitoren von MMP 13 sein können.

In dem Bestreben, wirksame Verbindungen zur Behandlung von Bindegewebserkrankungen zu finden, wurde nun gefunden, dass die erfindungsgemäß eingesetzten Verbindungen starke Inhibitoren der Matrix-Metalloproteinase 13 sind, während die erfindungsgemäß eingesetzten Verbindungen im wesentlichen unwirksam sind bei den MMPs 3 und 8.

Die Erfindung betrifft daher die Verwendung von Verbindungen der Formel I

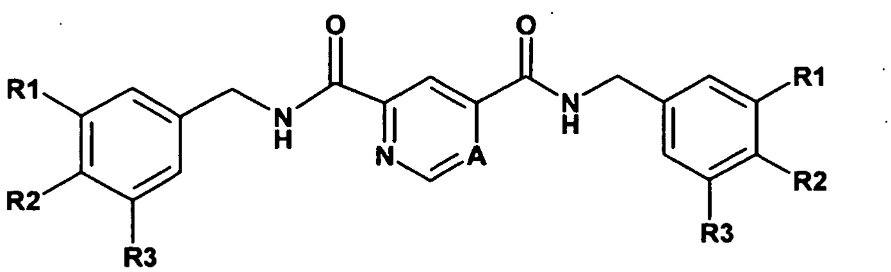

(I)

und/oder alle stereoisomere Formen der Verbindung der Formel I und/oder Gemische diese Formen in jedem Verhältnis, und/oder ein physiologisch verträgliches Salz der Verbindung der Formel I
zur Herstellung eines Arzneimittels zur Prophylaxe und Therapie von Spondylosen, Knorpelschwund nach Gelenktrauma oder längerer Gelenksruhigstellung nach Meniskus- oder Patellaverletzungen oder Bänderrissen, Kollagenosen, Arthropathien und Myalgien, wobei
A für ein Kohlenstoffatom oder Stickstoffatom steht,
R1 und R3 gleich oder verschieden sind und unabhängig voneinander für

1. Wasserstoffatom,
2. Halogen,
3. -($C_1$-$C_4$)-Alkyl, worin Alkyl unsubstituiert oder ein-, zwei- oder dreifach substituiert ist durch Halogen,
4. -O-($C_1$-$C_4$)-Alkyl, worin Alkyl unsubstituiert oder ein-, zwei- oder dreifach substituiert ist durch Halogen,
5. -C(O)-O-R4, worin R4 Wasserstoffatom oder -($C_1$-$C_4$)-Alkyl bedeutet,
6. -CN,
7. -N(R5)(R6), worin R5 und R6 gleich oder verschieden sind und unabhängig voneinander

    1. Wasserstoffatom,
    2. -($C_1$-$C_4$)-Alkyl,

3. $-C(O)-(C_1-C_4)$-Alkyl oder
4. $-SO_2-(C_1-C_4)$-Alkyl bedeuten,

8. -OH,
9. $-S-(C_1-C_4)$-Alkyl,
10. $-S(O)-(C_1-C_4)$-Alkyl,
11. $-S(O)_2$-R7, worin R7 $-(C_1-C_4)$-Alkyl, -OH oder $-NH_2$ bedeutet, stehen,

R2 für

1. Wasserstoffatom,
2. Halogen,
3. $-O-(C_1-C_4)$-Alkyl,
4. $-(C_1-C_4)$-Alkyl,
5. -C(O)-O-R4, worin R4 Wasserstoffatom oder $-(C_1-C_4)$-Alkyl bedeutet,
6. -CN,
7. -N(R5)(R6), worin R5 und R6 gleich oder verschieden sind und unabhängig voneinander

    1. Wasserstoffatom,
    2. $-(C_1-C_4)$-Alkyl,
    3. $-C(O)-(C_1-C_4)$-Alkyl oder
    4. $-SO_2-(C_1-C_4)$-Alkyl bedeuten,

8. -OH,
9. $-S-(C_1-C_4)$-Alkyl,
10. $-S(O)-(C_1-C_4)$-Alkyl,
11. $-S(O)_2$-R7, worin R7 $-(C_1-C_4)$-Alkyl, -OH oder $-NH_2$ bedeutet, steht oder R1 und R2 oder R2 und R3 zusammen mit den Kohlenstoffatomen an die sie jeweils gebunden sind einen 5- oder 6-gliedrigen Ring bilden, der aromatisch oder gesättigt ist und Null, ein oder zwei Heteroatome aus der Reihe Sauerstoff, Stickstoff oder Schwefel enthält und der andere Rest R1 oder R3 die obengenannte Bedeutung von 1. bis 11. hat.

Ein weiterer Gegenstand der Erfindung ist die Verwendung von Verbindungen der Formel I, wobei
A für ein Kohlenstoffatom oder Stickstoffatom steht,
R1 und R3 gleich oder verschieden sind und unabhängig voneinander für

1. Wasserstoffatom,
2. Halogen,
3. $-(C_1-C_4)$-Alkyl, worin Alkyl unsubstituiert oder ein-, zwei- oder dreifach substituiert ist durch Halogen,
4. $-O-(C_1-C_4)$-Alkyl, worin Alkyl unsubstituiert oder ein-, zwei- oder dreifach substituiert ist durch Halogen, stehen,

R2 für

1. Wasserstoffatom,
2. Halogen,
3. $-O-(C_1-C_4)$-Alkyl oder
4. $-(C_1-C_4)$-Alkyl steht, oder

R1 und R2 oder R2 und R3 zusammen mit den Kohlenstoffatomen an die sie jeweils gebunden sind einen 5- oder 6-gliedrigen Ring bilden, der aromatisch oder gesättigt ist und Null, ein oder zwei Heteroatome aus der Reihe Sauerstoff, Stickstoff oder Schwefel enthält und
der andere Rest R1 oder R3 die obengenannte Bedeutung von 1. bis 4. hat.
Ein weiterer Gegenstand der Erfindung ist die Verwendung von Verbindungen der Formel I, wobei
A für ein Kohlenstoffatom oder Stickstoffatom steht,
R1 und R3 gleich oder verschieden sind und unabhängig voneinander für

1. Wasserstoffatom,
2. Chlor,
3. Fluor,

4. Trifluormethyl,
5. Methoxyl,
6. Methyl,
7. -C(O)-OH,
8. $-C(O)-O-CH_3$,
9. -CN,
10. $-NH_2$,
11. $-NH-C(O)-CH_3$,
12. $-NH-SO_2-CH_3$,
13. $-N-(CH_3)_2$,
14. $-SO_2-NH_2$,
15. -OH,
16. $-O-CH_2-(CHF_2)$,
17. $-S-CH_3$,
18. $-S(O)-CH_3$,
19. $-S(O)_2-CH_3$ oder
20. Brom, stehen,

R2 für

1. Wasserstoffatom,
2. Chlor,
3. Fluor,
4. Methoxyl,
5. Methyl,
6. Brom,
7. -C(O)-OH,
8. $-C(O)-O-CH_3$,
9. -CN,
10. $-NH_2$,
11. $-NH-C(O)-CH_3$,
12. $-NH-SO_2-CH_3$,
13. $-N-(CH_3)_2$,
14. $-SO_2-NH_2$,
15. -OH,
16. $-O-CH_2-(CHF_2)$,
17. $-S-CH_3$,
18. $-S(O)-CH_3$ oder
19. $-S(O)_2-CH_3$, steht, oder

R1 und R2 oder R2 und R3 zusammen mit den Kohlenstoffatomen an die sie jeweils gebunden sind einen Dioxolan-, Dihydrofuran- oder Furan-Ring bilden und
der andere Rest R1 oder R3 die obengenannte Bedeutung von 1. bis 20. hat.

Ein weiterer Gegenstand der Erfindung ist die Verwendung von Verbindungen der Formel I, wobei
A für ein Kohlenstoffatom oder Stickstoffatom steht,
R1 und R3 gleich oder verschieden sind und unabhängig voneinander für

1. Wasserstoffatom,
2. Chlor,
3. Fluor,
4. Trifluormethyl,
5. Methoxyl, oder
6. Methyl, stehen,

R2 für

1. Wasserstoffatom,
2. Chlor,

3. Fluor,
4. Methoxyl oder
5. Methyl steht, oder

R1 und R2 oder R2 und R3 zusammen mit den Kohlenstoffatomen an die sie jeweils gebunden sind einen Dioxolan-, Dihydrofuran- oder Furan-Ring bilden.

Ein weiterer Gegenstand der Erfindung sind Verbindungen der Formel I,

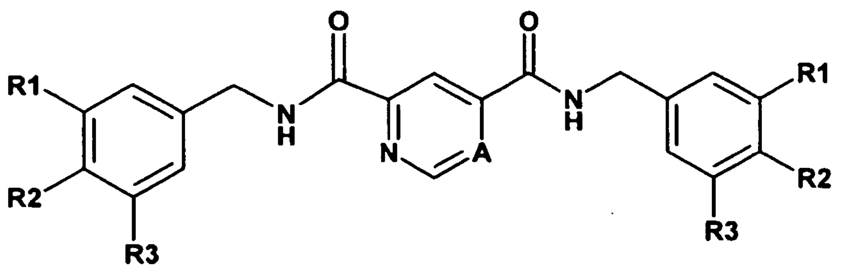

(I)

und/oder alle stereoisomere Formen der Verbindung der Formel I und/oder Gemische diese Formen in jedem Verhältnis, und/oder ein physiologisch verträgliches Salz der Verbindung der Formel I, wobei
A für ein Stickstoffatom steht,
R1 und R3 gleich oder verschieden sind und unabhängig voneinander für

1. Wasserstoffatom,
2. -($C_1$-$C_4$)-Alkyl, worin Alkyl ein-, zwei- oder dreifach substituiert ist durch Halogen, oder
3. -O-($C_1$-$C_4$)-Alkyl, worin Alkyl ein-, zwei- oder dreifach substituiert ist durch Halogen, stehen, und

R2 für

1. Wasserstoffatom,
2. Halogen,
3. -O-($C_1$-$C_4$)-Alkyl oder
4. -($C_1$-$C_4$)-Alkyl steht,

mit Ausnahme des Falles, dass

a) die Reste R1, R2 und R3 alle gleichzeitig Wasserstoffatom sind oder
b) alle Reste R1, R2 und R3 unabhängig voneinander nur Wasserstoffatom, Halogen, -($C_1$-$C_4$)-Alkyl oder -($C_1$-$C_4$)-Alkoxy bedeuten, oder

R1 und R2 oder R2 und R3 zusammen mit den Kohlenstoffatomen an die sie jeweils gebunden sind einen Dihydrofuran-Ring bilden und
der andere Rest R1 oder R3 für

1. Wasserstoffatom,
2. Halogen,
3. -($C_1$-$C_4$)-Alkyl, worin Alkyl unsubstituiert oder ein-, zwei- oder dreifach substituiert ist durch Halogen, oder
4. -O-($C_1$-$C_4$)-Alkyl, worin Alkyl ein-, zwei- oder dreifach substituiert ist durch Halogen, steht.

Ein weiterer Gegenstand der Erfindung sind Verbindungen der Formel I, wobei
A für ein Stickstoffatom steht,
R1 und R3 gleich oder verschieden sind und unabhängig voneinander für

1. Wasserstoffatom oder
2. Trifluormethyl, stehen,

worin R1, R2 und R3 nicht gleichzeitig Wasserstoffatom sind, und

R2 für

1. Wasserstoffatom,
2. Chlor,
3. Fluor,
4. Methoxyl oder
5. Methyl steht, oder

R1 und R2 oder R2 und R3 zusammen mit den Kohlenstoffatomen an die sie jeweils gebunden sind einen Dihydrofuran-Ring bilden und
der andere Rest R1 oder R3 für

1. Wasserstoffatom,
2. Chlor,
3. Fluor, .
4. Trifluormethyl oder
5. Methyl steht.

Unter dem Begriff "Halogen" wird Fluor, Chlor, Brom oder Jod verstanden. Unter dem Begriff "($C_1$-$C_4$)-Alkyl" werden Kohlenwasserstoffreste verstanden, deren Kohlenstoffkette geradkettig oder verzweigt ist und 1 bis 4 Kohlenstoffatome enthält, beispielsweise Methyl, Ethyl, Propyl, i-Propyl, Butyl, oder tertiär-Butyl.
Unter dem Begriff "R1 und R2 oder R2 und R3 zusammen mit den Kohlenstoffatomen an die sie jeweils gebunden sind einen 5- oder 6-gliedrigen Ring bilden, der aromatisch oder gesättigt ist und Null, ein oder zwei Heteroatome aus der Reihe Sauerstoff, Stickstoff oder Schwefel enthält" werden Reste verstanden die sich von Dioxolan, Pyrrol, Pyrrolidin, Pyridin, Piperidin, Tetrahydropyridin, Pyrazol, Imidazol, Pyrazolin, Imidazolin, Pyrazolidin, Imidazolidin, Pyridazin, Pyrimidin, Pyrazin, Piperazin, Pyran, Furan, Dihydrofuran, Tetrahydrofuran, Oxazol, Isoxazol, 2-Isoxazolin, Isoxazolidin, Morpholin, Oxothiolan, Thiopyran, Thiazol, Isothiazol, 2-Isothiazolin, Isothiazolidin oder Thiomorpholin ableiten lassen.
Die Verbindungen der Formel I lassen sich beispielsweise dadurch herstellen, dass man eine Verbindung der Formel II

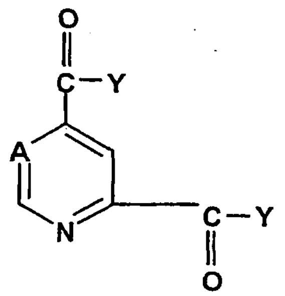

(II)

a) mit einer Verbindung der Formel III

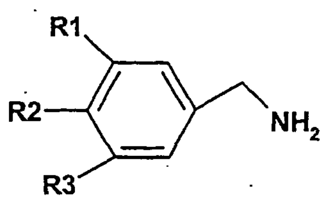

III

umsetzt, wobei R1, R2 und R3 die in Formel 1 angegebenen Bedeutungen haben und Y Halogen, Hydroxy oder $C_1$-$C_4$-Alkoxy ist oder zusammen mit der Carbonylgruppe einen Aktivester oder ein gemischtes Anhydrid bildet, wobei eine Verbindung der Formel I gebildet wird und die Reaktionsprodukte gegebenenfalls in ihre physiologisch verträglichen Salze überführt, oder
b) eine Verbindung der Formel II mit einer Verbindung der Formel III zu einer Verbindung der Formel IV.

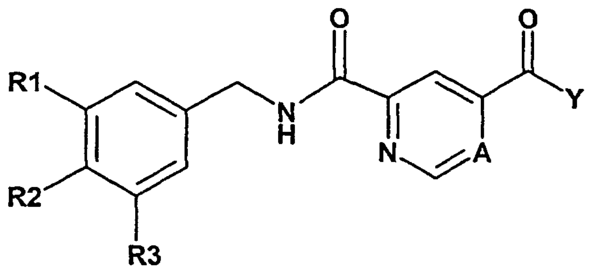

(IV)

umsetzt, wobei R1, R2 und R3 die in Formel 1 angegebenen Bedeutungen haben und Y Halogen, Hydroxy oder $C_1$-$C_4$-Alkoxy ist oder zusammen mit der Carbonylgruppe einen Aktivester oder ein gemischtes Anhydrid bildet und die Verbindung der Formel IV gegebenenfalls reinigt und anschließend mit einer Verbindung der Formel III in eine Verbindung der Formel I überführt.

Im Folgenden wird die Herstellung von Verbindungen gemäß Formel I und die Herstellung der dafür benötigten Ausgangssubstanzen, sofern sie nicht käuflich sind, näher beschrieben.

Die Herstellung der erfindungsgemäßen Verbindungen gelingt am einfachsten dadurch, dass die beiden Komponenten, das Pyrimidin- oder Pyridin-Derivat gemäß Formel (II) und das Amin gemäß Formel (III) in äquimolaren Mengen oder bis zu einem etwa 5-fachen Überschuss an III zusammengegeben werden und bei Temperaturen zwischen -30°C bis 150 °C, bevorzugt bei 20 °C bis 100°C bis zur Beendigung der Reaktion umgesetzt werden. Bei der Herstellung der Verbindung der Formel IV wird das Amin gemäß Formel (III) bis zu einer äquimolaren Menge an der Verbindung der Formel III zusammengegeben und wie oben umgesetzt. Die Beendigung der Reaktion lässt sich beispielsweise mittels Dünnschicht-chromatographie oder HPLC-MS bestimmen. Eine Variante dieses Verfahrens besteht darin, dass man in einem geeigneten Lösungsmittel, wie Diethylether, Dimethoxyethan oder Tetrahydrofuran, chlorierten Kohlenwasserstoffen wie Methylenchlorid, Chloroform, Tri- oder Tetrachlorethylen, Benzol, Toluol oder auch polaren Lösungsmitteln wie Dimethylformamid, Aceton oder Dimethylsulfoxid arbeitet. Auch hier kann ein Überschuss von Amin gemäß Formel (III), der bis zur etwa 5-fachen Mengen betragen kann, angewandt werden. Die Reaktionstemperaturen liegen dabei zwischen Raumtemperatur und dem Siedepunkt des Lösungsmittels, wobei Temperaturen im Bereich von Raumtemperatur bis 130 ° C besonders bevorzugt sind.

Ebenso kann die Umsetzung über ein gemischtes Anhydrid wie Chlorameisensäure-ethylester oder über einen aktiven Ester wie Paranitrophenylester (Y = $ClCH_2$-COO oder $NO_2$-$C_6H_4$-O) erfolgen. Entsprechende Methoden sind in der Literatur beschrieben.

Gegebenenfalls kann die Umsetzung auch in Gegenwart von Basen erfolgen. Als zusätzliche Basen kommen beispielsweise Carbonate oder Hydrogencarbonate wie Natrium- oder Kaliumcarbonat oder Natrium- oder Kaliumhydrogencarbonat, oder tertiäre Amine, wie Triethylamin, Tributylamin, Ethyldiisopropylamin oder heterocyclische Amine wie N-Alkylmorpholin, Pyridin, Chinolin oder Dialkylaniline in Betracht.

Gegebenenfalls kann die Aufarbeitung der Produkte, insbesondere die Verbindung der Formel IV, beispielsweise durch Extraktion oder Chromatographie z. B. über Kieselgel erfolgen. Das isolierte Produkt kann umkristallisiert und gegebenenfalls mit einer geeigneten Säure zu einem physiologisch verträglichen Salz umgesetzt werden. Als geeignete Säuren kommen beispielsweise in Betracht:

Mineralsäuren, wie Chlorwasserstoff- und Bromwasserstoffsäure sowie Schwefel-, Phosphor-, Salpeter- oder Perchlorsäure oder organische Säuren wie Ameisen-, Essig-, Propion-, Bernstein-, Glykol-, Milch-, Äpfel-, Wein-, Zitronen-, Malein-, Fumar-, Phenylessig-, Benzoe-, Methansulfon-, Toluolsulfon-, Oxal-, 4-Aminobenzoe-, Naphthalin-1,5-disulfon- oder Ascorbinsäure.

Die Ausgangsverbindungen der Formel (III) können, soweit sie nicht käuflich sind, einfach synthetisiert werden (z. B. Organikum, Organisch Chemisches Grundpraktikum, 15. Aufl., VEB Deutscher Verlag der Wissenschaften, 1976; eine Übersicht über die verschiedenen Möglichkeiten findet sich im Methodenregister, S. 822).

Die Ausgangsverbindungen der Formel (II) erhält man beispielsweise durch Umsetzung von Pyrimidin-4,6-dicarbonsäure, bzw. Pyridin-2,4-dicarbonsäure, zu dem entsprechenden Pyrimidin-4,6-dicarbonsäurehalogenid bzw. Pyridin-2,4-dicarbonsäurehalogenid, bevorzugtchlorid (nach literaturbekannten Verfahren), vorzugsweise in Gegenwart eines Katalysators wie Dimethylformamid. Dieses Säurehalogenid kann dann beispielsweise entweder mit einem geeigneten Alkohol, z. B. Paranitrobenzylalkohol zu dem entsprechenden Aktivester, oder aber mit niederen Alkoholen wie Methanol oder Ethanol zu den entsprechenden Estern umgesetzt werden. Ebenso kann die Pyrimidin-4,6-dicarbonsäure auch zunächst unter Zusatz einer geeigneten Carbonsäure oder eines Carbonsäureesters wie Chlorameisensäureäthylester in ein gemischtes Anhydrid überführt werden, welches dann mit den Aminen der Verbindung der Formeln (III) und (IV) zu den erfindungsgemäßen Produkten umgesetzt wird. Eine entsprechende Methode ist ebenfalls in der Literatur beschrieben.

Die Herstellung der Pyrimidin-4,6-dicarbonsäure erfolgt nach literaturbekannten Verfahren, beispielsweise durch Oxidation von 4,6-Dimethylpyrimidin, welches seinerseits beispielsweise erhältlich ist durch katalytische Hydrierung von käuflich erhältlichem 2-Mercapto-4,6-dimethylpyrimidin.

Sofern Verbindungen der Formel I diastereoisomere oder enantiomere Formen zulassen und bei der gewählten Synthese als deren Gemische anfallen, gelingt die Trennung in die reinen Stereoisomeren entweder durch Chromatographie an einem gegebenenfalls chiralen Trägermaterial, oder, sofern die racemische Verbindung der Formel I zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L- und D-Arginin die unterschiedlich löslichen diastereomeren Salze gebildet, die schwerer lösliche Komponente als Feststoff isoliert, das leichter lösliche Diastereomer aus der Mutterlauge abgeschieden, und aus den so gewonnenen diastereomeren Salzen die reinen Enantiomeren gewonnen. Auf prinzipiell gleiche Weise kann man die racemischen Verbindungen der Formel 1, die eine basische Gruppe wie eine Aminogruppe enthalten, mit optisch aktiven Säuren, wie (+)-Campher-10-sulfonsäure, D- und L- Weinsäure, D- und L- Milchsäure sowie (+) und (-)-Mandelsäure in die reinen Enantiomeren überführen. Auch kann man chirale Verbindungen, die Alkohol- oder Aminfunktionen enthalten, mit entsprechend aktivierten oder gegebenenfalls N- geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der isomeren genützt werden, indem man eine Trennung der nunmehr vorliegenden Diastereomeren durch Kristallisation oder Chromatographie an geeigneten Stationärphasen vornimmt und danach den mitgeführten chiralen Molekülteil mittels geeigneter Methoden wieder abspaltet.

Saure oder basische Produkte der Verbindung der Formel I können in Form ihrer Salze oder in freier Form vorliegen. Bevorzugt sind pharmakologisch verträgliche Salze, z. B. Alkali- oder Erdalkalimetallsalze bzw. Hydrochloride, Hydrobromide, Sulfate, Hemisulfate, alle möglichen Phosphate sowie Salze der Aminosäuren, natürlicher Basen oder Carbonsäuren.

Die Herstellung physiologisch verträglicher Salze aus zur Salzbildung befähigten Verbindungen der Formel I, einschließlich deren stereoisomeren Formen, erfolgt in an sich bekannter Weise. Die Carbonsäuren und Hydroxamsäuren bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Ornithin oder Arginin, stabile Alkali-, Erdalkali oder gegebenenfalls substituierte Ammoniumsalze. Sofern die Verbindungen der Formel I basische Gruppen aufweist, lassen sich mit starken Säuren auch stabile Säureadditionssalze herstellen. Hierfür kommen sowohl anorganische als auch organische Säuren, wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamido-sulfon-, Trifluormethylsulfon-, Essig-, Oxal-, Wein-, Bernstein- oder Trifluoressigsäure in Frage.

Aufgrund der pharmakologischen Eigenschaften eignen sich die Verbindungen der Formel I zur Prophylaxe und Therapie all solcher Erkrankungen, an deren Verlauf eine verstärkte Aktivität von Matrix-Metalloproteinase 13 beteiligt ist.

Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels oder Krebserkrankungen wie Brustkrebs.

Die Applikation der erfindungsgemäßen Arzneimittel kann durch subkutane, intraartikuläre, intraperitoneale oder intravenöse Injektion erfolgen. Bevorzugt ist die intraartikuläre Injektion. Die rektale, orale, inhalative oder transdermale Applikation ist auch möglich.

Die Erfindung betrifft auch ein Verfahren zur Herstellung eines Arzneimittels, das dadurch gekennzeichnet, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Die Verbindungen der Formel 1 werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wässrige alkoholische oder ölige Suspensionen oder wässrige oder ölige Lösungen. Als inerte Trägerstoffe können z. B. Gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen, intraartikulären, intraperitonealen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage physiologische Kochsalzlösung oder Alkohole, z. B. Ethanol, Propanol, Glycerin, daneben auch Zuckerlösungen wie Glukose- oder Mannitlösungen, oder auch eine Mischung aus den verschiedenen genannten Lösungsmitteln.

Ferner finden übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Die Verbindungen der Formel 1 werden bevorzugt als pharmazeutische Präparate in Dosierungseinheiten hergestellt und verabreicht, wobei jede Einheit als aktiven Bestandteil eine bestimmte Dosis der Verbindung der Formel I enthält. Sie können zu diesem Zweck oral in Dosen von 0,01 mg/kg/Tag bis 25,0 mg/kg/Tag, vorzugsweise 0,01 mg/kg/Tag bis 5,0 mg/kg/Tag oder parenteral in Dosen von 0,001 mg/kg/Tag bis 5 mg/kg/Tag, vorzugsweise 0,001 mg/kg/Tag bis 2,5 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Nachfolgend ist die Erfindung an Hand von Beispielen näher erläutert.

Beispiel 1:

Pyrimidin-4,6-dicarbonsäure-dibenzylamid (Formel I: R1 = R2 = H)

1,7 g Pyrimidin-4,6-dicarbonsäure werden in 20 ml Toluol suspendiert und 2,4 g Thionylchlorid und 0,2 ml Dimethylformamid zugegeben. Der Ansatz wird zum Rückfluss erhitzt, bis keine Gasentwicklung mehr zu beobachten ist (etwa 3 Stunden (h)). Etwa 5 ml Lösungsmittel werden abdestilliert, der Ansatz auf 0 ˚C bis 10 ˚C abgekühlt und mit 2,7 g Benzylamin, gelöst in 10 ml Toluol, versetzt. Die Lösung wird langsam auf Raumtemperatur erwärmt, 12 Stunden bei Raumtemperatur gerührt und zur Trockene eingedampft. Der Rückstand wird mit 50 ml Methylenchlorid aufgenommen, 3 mal mit gesättigter Natriumhydrogencarbonat-Lösung ausgeschüttelt, die organische Phase mit Wasser gewaschen, mit Magnesiumsulfat getrocknet und eingedampft.
Der Festkörper wird aus Diisopropylether umkristallisiert.
Ausbeute: 2,1 g; Fp.: 131 ˚C bis 132 ˚C.

Beispiel 2:

Pyrimidin-4,6-dicarbonsäure bis-(3-chloro-4-fluoro-benzylamid) (Formel I: R1 = Cl; R2 = F)

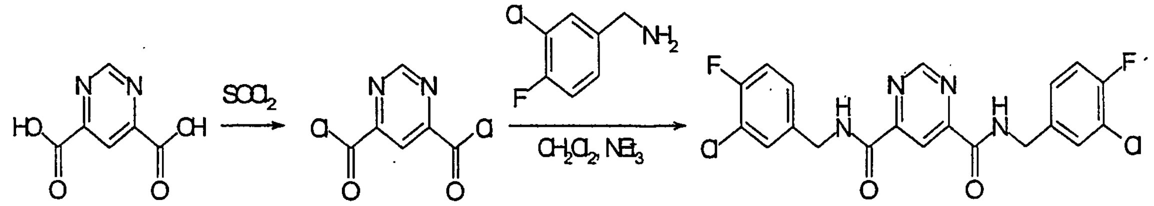

200 mg (1,2 mmol) Pyrimidin-4,6-dicarbonsäure wurden in 0,3 ml (4,1 mmol) Thionylchlorid suspendiert. Diese Mischung wurde unter Rühren für 2 h auf 85 ˚C geheizt. Nach dem Abkühlen auf Raumtemperatur wurden 2 ml absolutes Dichlormethan zugegeben. Die Suspension wurde auf 0 ˚C gekühlt und 0,33 ml (2,4 mmol) Triethylamin wurden zugesetzt. 861 mg (5,4 mmol) 3-Chloro-4-fluorobenzyl-amin wurden unter heftigem Rühren zugefügt. Die Mischung wurde weitere 15 Minuten gerührt. Dann wurde mit 10 ml Dichlormethan verdünnt und 10 ml Wasser zugesetzt. Nach 5 Minuten wurde die Mischung in einen Scheidetrichter überführt und die Phasen wurden getrennt. Die organische Phase wurde zweimal mit gesättigter Kochsalzlösung extrahiert und nachfolgend über Magnesiumsulfat getrocknet. Nach der Filtration wurde das Filtrat unter verminderten Druck eingeengt und der so erhaltene Rückstand wurde in Ethylacetat gelöst. Durch Zugabe von Heptan wurde das Produkt aus der Lösung kristallisiert. Beigefarbene Blättchen wurden erhalten und unter verminderten Druck getrocknet. Ausbeute: 263 mg (49 %)

Analog zu Beispiel 2 wurden die nachfolgenden Verbindungen hergestellt.

Tabelle 1:

| Beispiel | Struktur | MS (ESI+) |
|---|---|---|
| 3 | 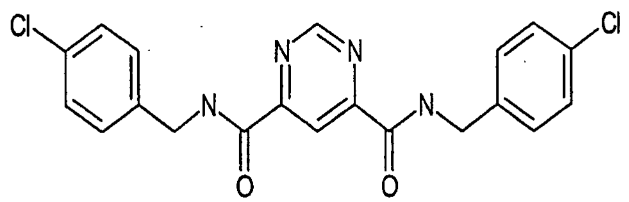 | 415,13 |
| 4 | 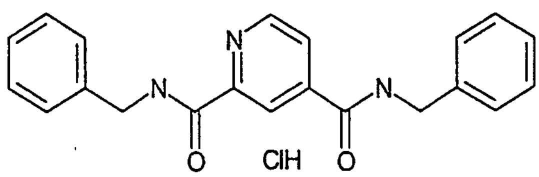 | 346,27 |
| 5 | 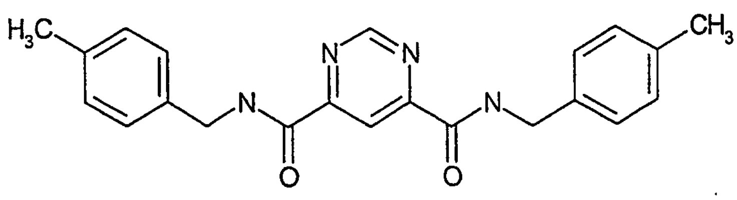 | 375,26 |
| 6 | 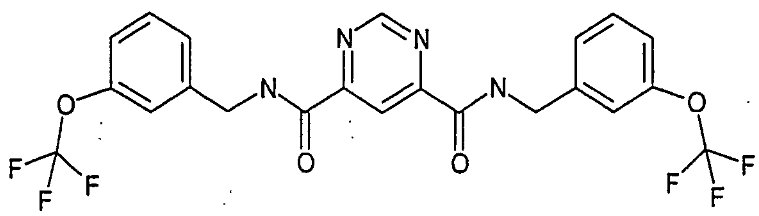 | 515,21 |
| 7 | 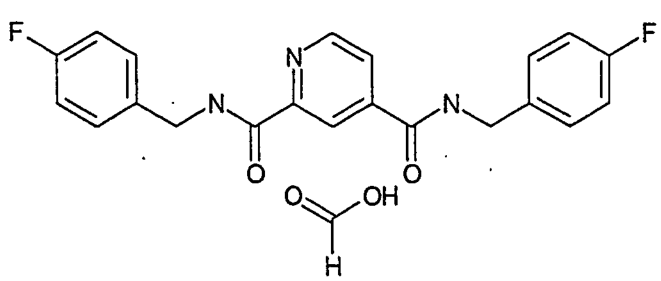 | 382,21 |

(fortgesetzt)

| Beispiel | Struktur | MS (ESI+) |
|---|---|---|
| 8 | 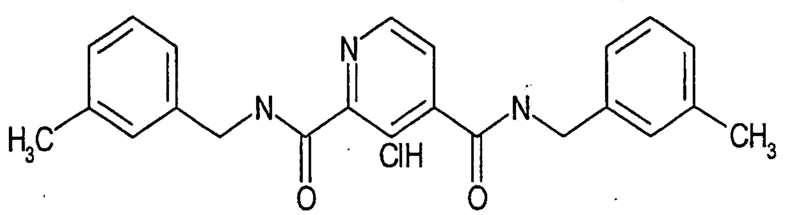 | 374,25 |
| 9 | 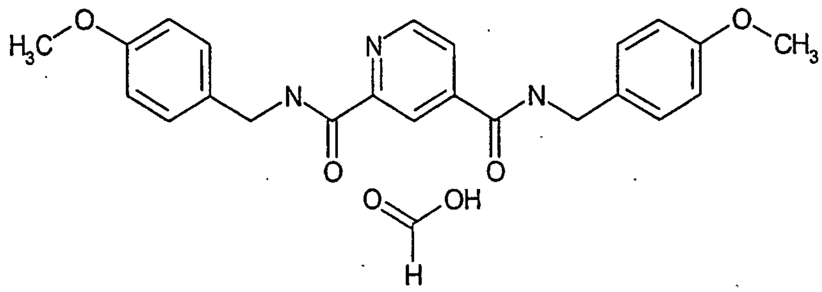 | 406,31 |
| 10 | 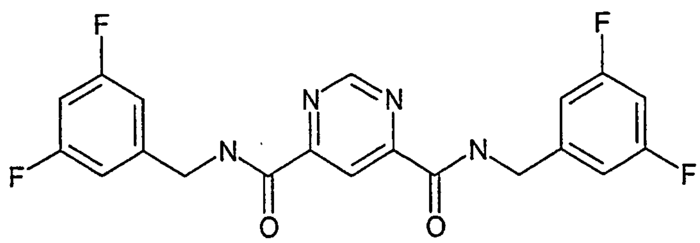 | 419,22 |
| 11 | 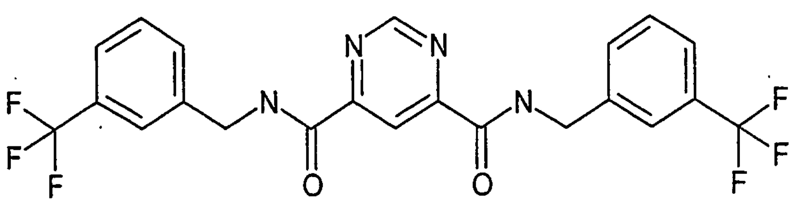 | 483,24 |
| 12 | 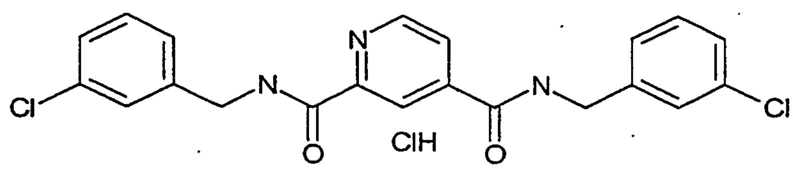 | 414,15 |
| 13 | 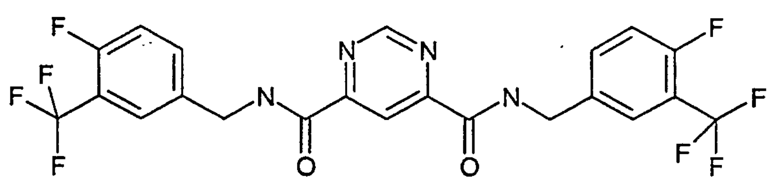 | 560,18 (M+MeCN) |
| 14 | 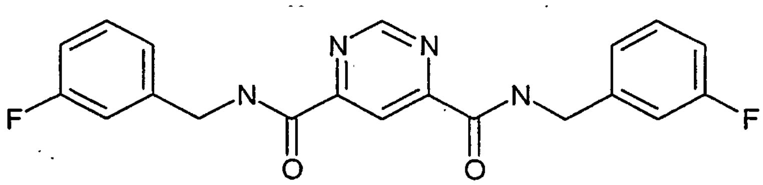 | 383,17 |

(fortgesetzt)

| Beispiel | Struktur | MS (ESI+) |
|---|---|---|
| 15 | 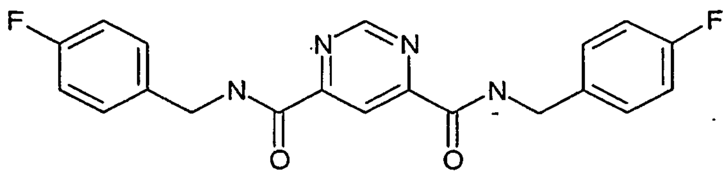 | 383,15 |
| 16 | 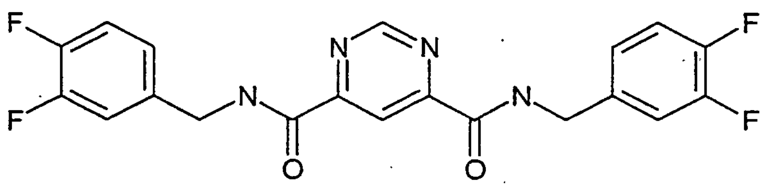 | 419,02 |
| 17 | 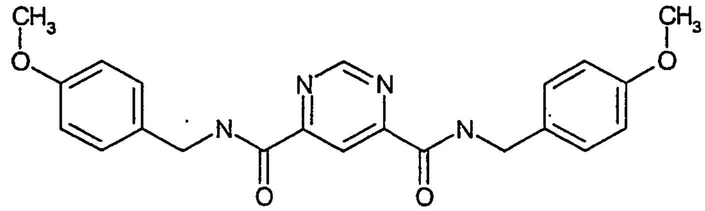 | 407,23 |
| 18 | 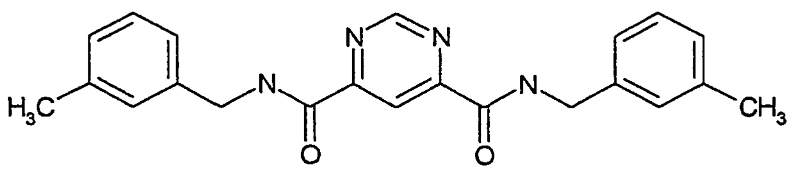 | 375,13 |
| 19 | 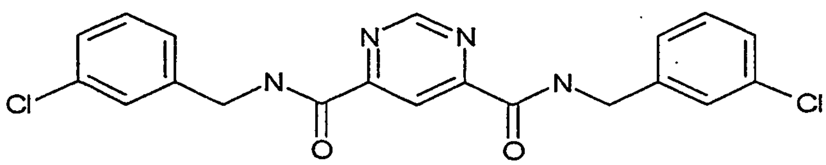 | 415,06 |
| 20 | 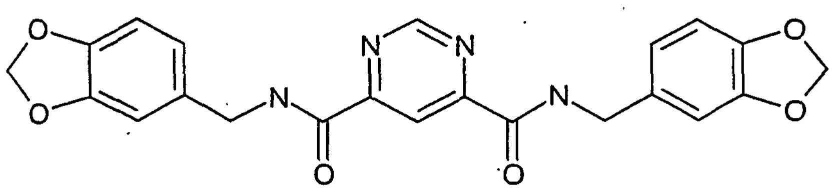 | 435,22 |
| 21 | 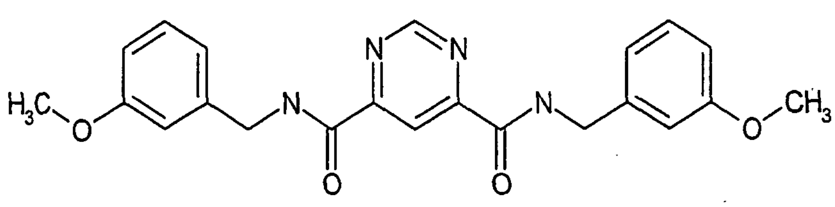 | 407,30 |

(fortgesetzt)

| Beispiel | Struktur | MS (ESI+) |
|---|---|---|
| 22 | 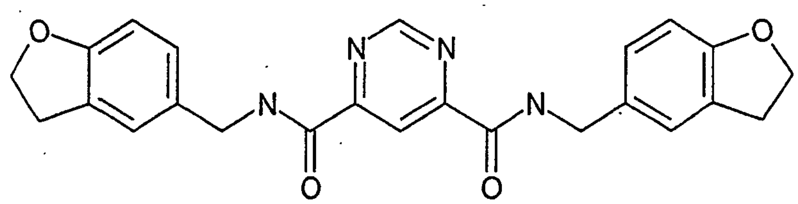 | 431,06 |
| 23 | 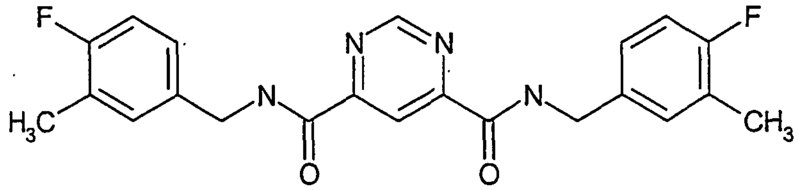 | 411,25 |

Beispiel 24 Pyrimidin-4,6-dicarbonsäuredimethylester

10 g (0,059 mol) Pyrimidin-4,6-dicarbonsäure wurde in 1,4 L Methanol suspendiert, mit 10,93 mL (0,356 mol) konzentrierter Salzsäure versetzt und 3 Stunden (h) am Rückfluss (65 ˚C) gerührt. Das Reaktionsgemisch wurde unter verminderten Druck eingeengt, nochmals in Methanol aufgenommen, filtriert und die erhaltene Lösung wurde eingeengt. Ausbeute 11,02 g (94,4 %) MS ($ES^+$): m/e= 197,20

2,55 g (0,01299 mol) der erhaltenen Verbindung Pyrimidin-4,6-dicarbonsäuredi-methylester wurden in 100 mL Dimethylformamid (DMF) gelöst, mit 1,42 mL (0,01299 mol) Benzylamin versetzt und auf 50 ˚C erwärmt. Nach 4 h wird die Lösung unter verminderten Druck eingeengt. Der Rückstand wird über eine 500 ml Kieselgelsäule mit Heptan/ Essigester (1:1) chromatographiert. Fraktionen mit der Verbindung 6-Benzylcarbamoyl-pyrimidin-4-carbonsäuremethylester wurden eingeengt.
Ausbeute: 1,268 g (36 %) MS ($ES^+$): m/e= 272,20

200 mg (0,737 mmol) der erhaltenen Verbindung 6-Benzylcarbamoyl-pyrimidin-4-carbonsäuremethylester wurden in 4 mL DMF gelöst, mit 225,98 mg (1,29 mmol) 3-Trifluormethyl-benzylamin versetzt und bei 50 ˚C 1 Tag gerührt. Danach wurde die Lösung unter verminderten Druck eingeengt. Der Rückstand wurde mittels präparativer HPLC (Wasser/Acetonitril-Gradient, Purospher RP18) gereinigt. Fraktionen mit Pyrimidin-4,6-dicarbonsäure-4-benzylamid-6-(3-trifluormethyl-benzylamid) wurden unter vermindertem Druck eingeengt und gefriergetrocknet. Ausbeute: 240 mg (79 %) MS ($ES^+$): m/e= 415,27

In analoger Weise wurden die folgenden Verbindungen hergestellt:

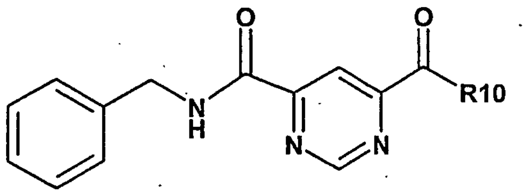

Tabelle 2

| Beispiel | Rest R10 | MS ($ES^+$): m/e |
|---|---|---|
| 25 | 3-Fluorbenzylamin | 365,23 |
| 26 | 4-Fluorbenzylamin | 365,23 |
| 27 | 3,4-Difluorbenzylamin | 383,27 |
| 28 | 4-Methoxybenzylamin | 377,28 |
| 29 | 3-Methylbenzylamin | 361,28 |

(fortgesetzt)

| Beispiel | Rest R10 | MS (ES+): m/e |
|---|---|---|
| 30 | 3-Chlorbenzylamin . | 381,23 |

In analoger Weise zu den Beispielen 1 bis 30 wurden die folgenden Beispiele hergestellt:

Tabelle 3

| Beispiel | Struktur | MS (ES+): m/e |
|---|---|---|
| 31 | 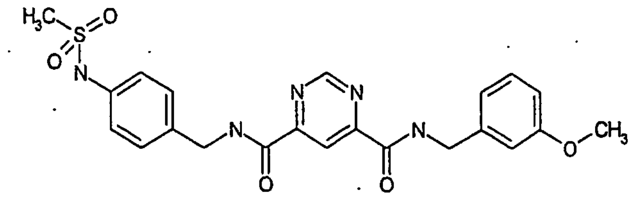 | 470,22 ES+ |
| 32 | 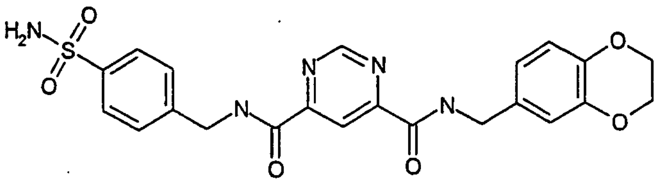 | 484,12 ES+ |
| 33 | 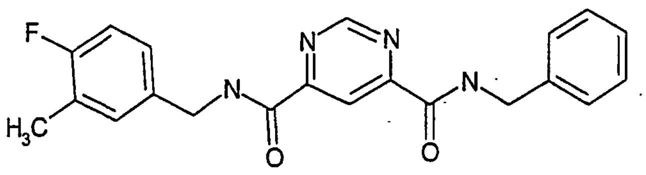 | 378,15 ES+ |
| 34 | 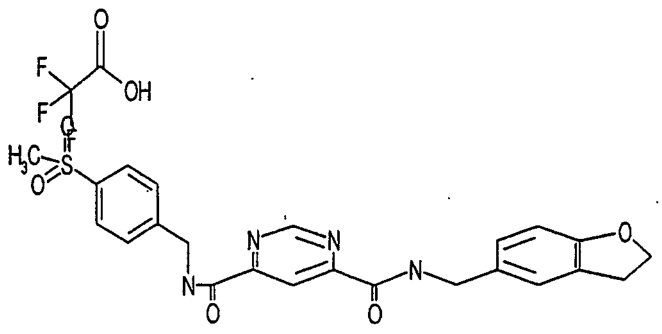 | 466,13 ES+ |
| 35 | 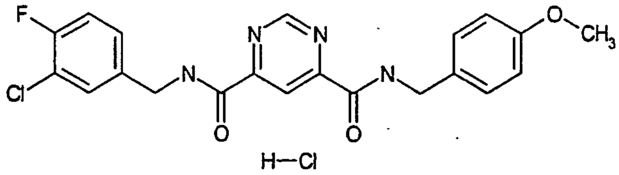 | 429,17 ES+ |
| 36 | 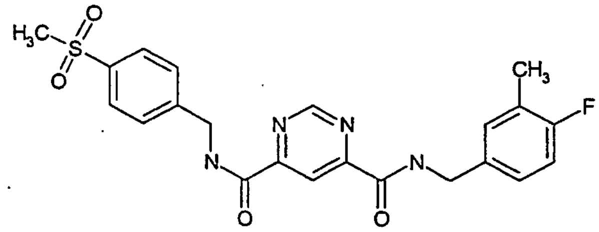 | 456,13 ES+ |

(fortgesetzt)

| Beispiel | Struktur | MS (ES+): m/e |
|---|---|---|
| 37 | 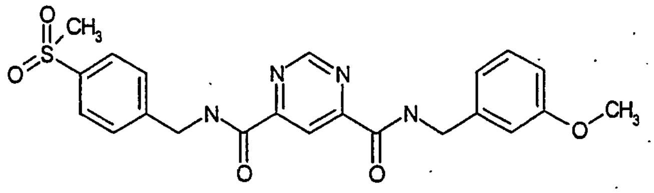 | 455,10 ES+ |
| 38 | 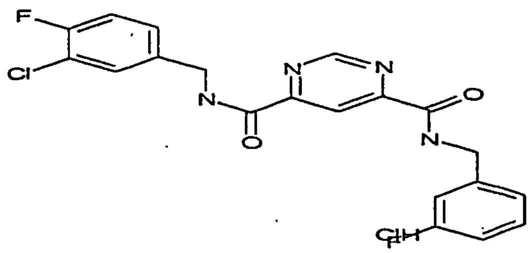 | 417,11 ES+ |
| 39 | 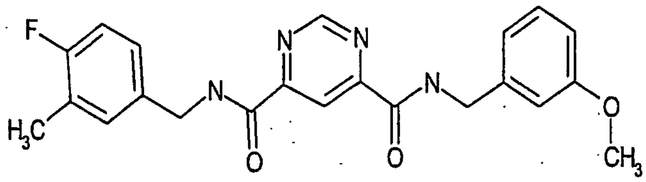 | 409,28 ES+ |
| 40 | 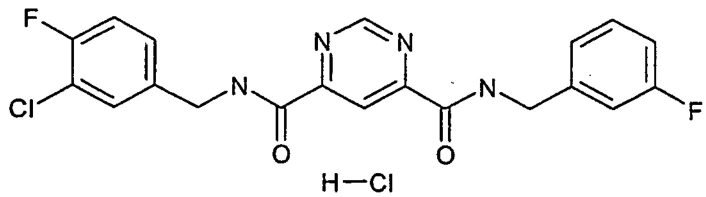 | 417,11 ES+ |
| 41 | 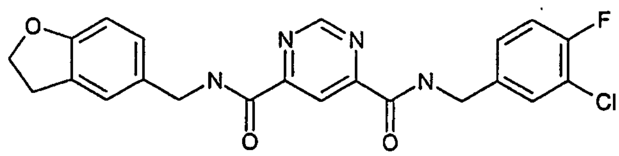 | 441,25 |
| 42 | 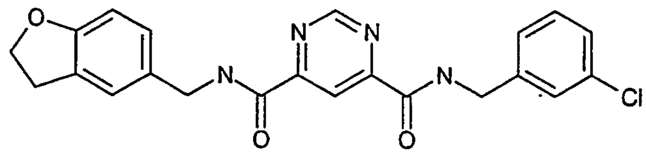 | 423,26 |
| 43 | 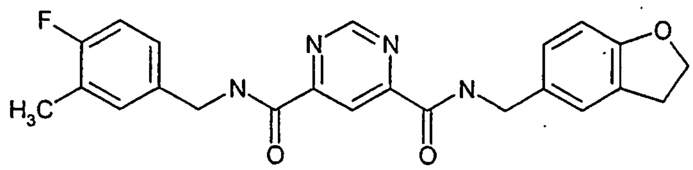 | 421,29 ES+ |
| 44 | 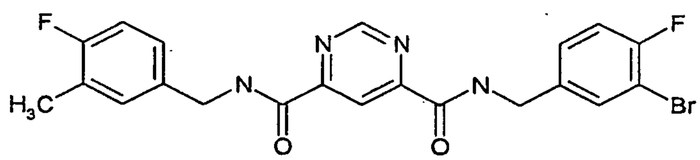 | 477,15 ES+ |

(fortgesetzt)

| Beispiel | Struktur | MS ($ES^+$): m/e |
|---|---|---|
| 45 | 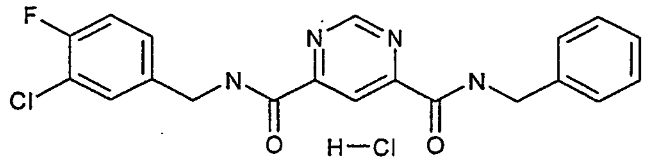 | 399,20 ES+ |
| 46 | 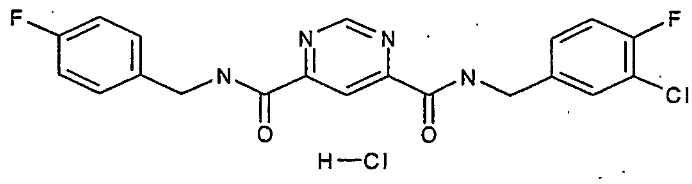 | 417,16 ES+ |
| 47 | 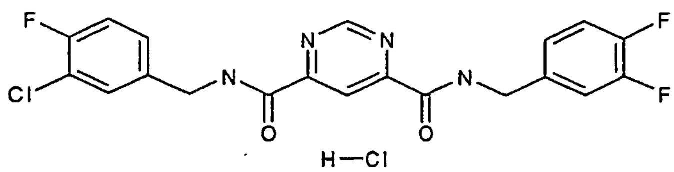 | 435,14 ES+ |
| 48 | 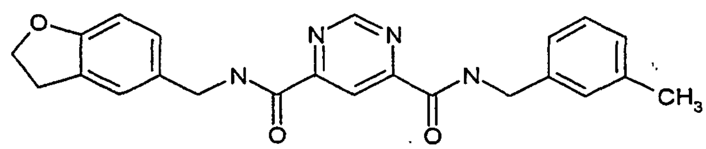 | 403,31 ES+ |
| 49 | 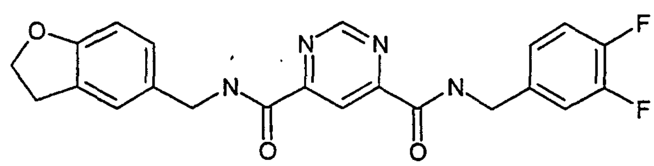 | 425,30 ES+ |
| 50 | 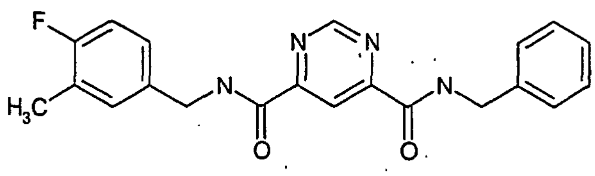 | 379,29 ES+ |
| 51 | 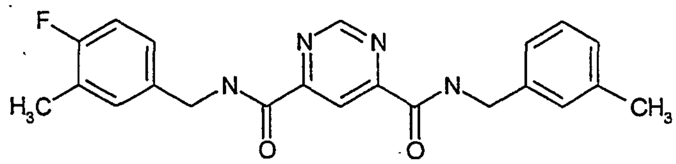 | 393,33 ES+ |
| 52 | 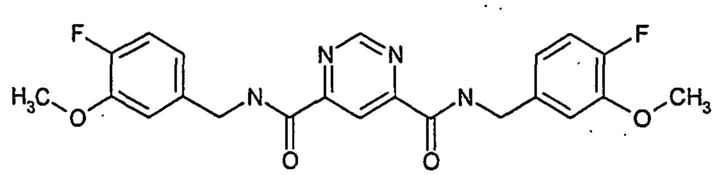 | 443,28 ES+ |

(fortgesetzt)

| Beispiel | Struktur | MS (ES+): m/e |
|---|---|---|
| 53 | 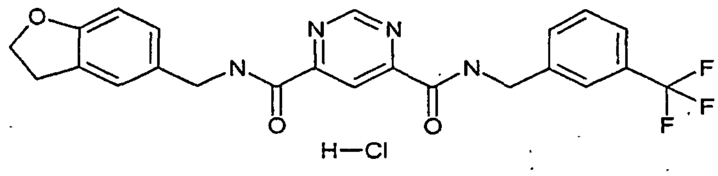 | 457,20 ES+ |
| 54 | 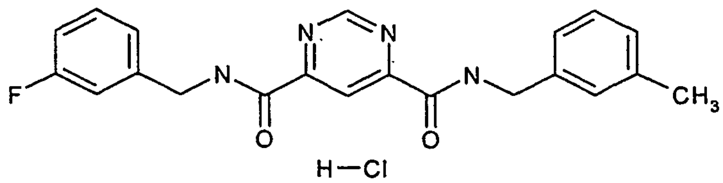 | 379,19 ES+ |
| 55 | 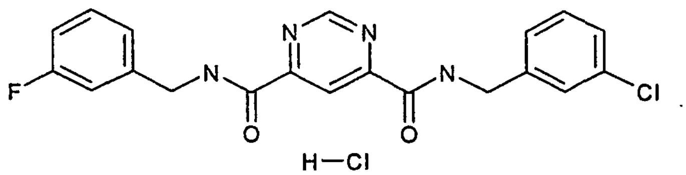 | 399,14.ES+ |
| 56 | 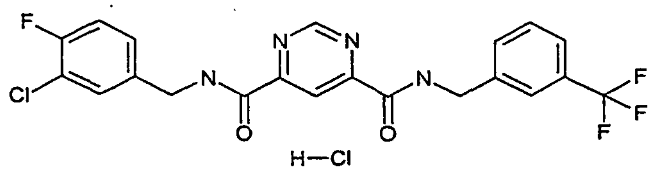 | 467,17 ES+ |
| 57 | 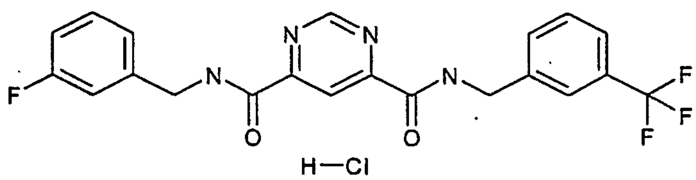 | 433,24 ES+ |
| 58 | 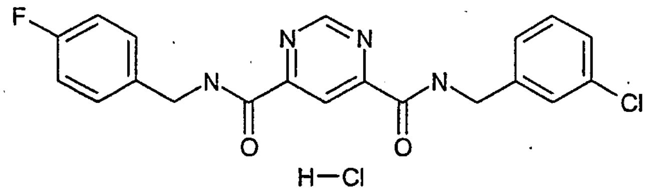 | 399,18 ES+ |
| 59 | 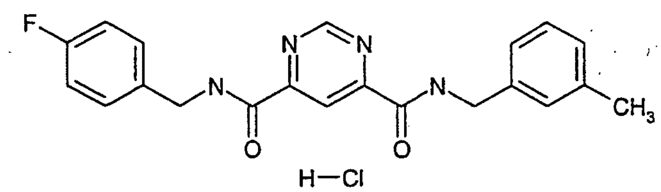 | 379,10 ES+ |
| 60 | 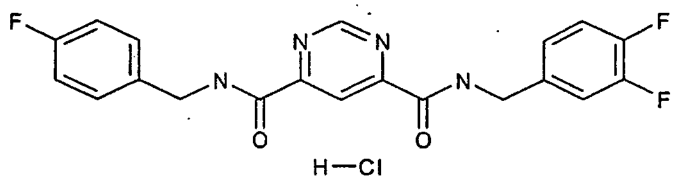 | 401,05 ES+ |

(fortgesetzt)

| Beispiel | Struktur | MS (ES+): m/e |
|---|---|---|
| 61 | 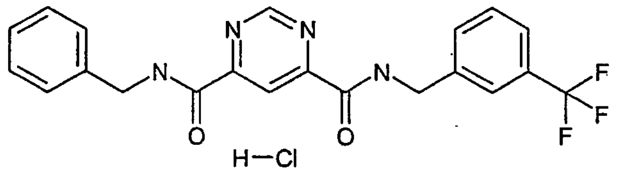 | 415,27 ES+ |
| 62 | 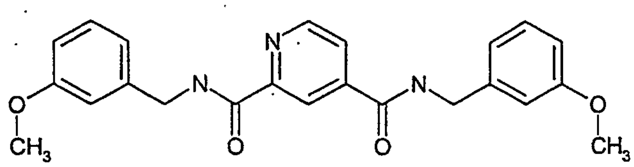 | 406,26 ES+ |
| 63 | 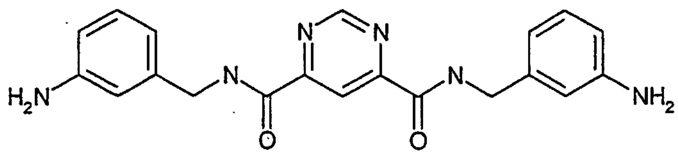 | 377,32 ES+ |
| 64 | 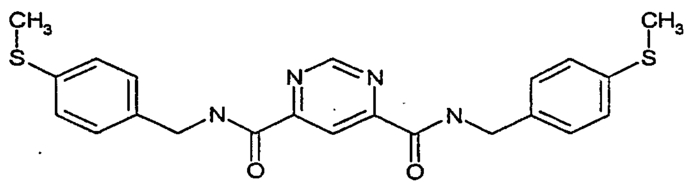 | 439,21 ES+ |
| 65 | 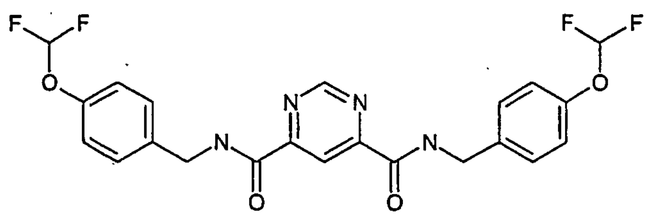 | 479,18 ES+ |
| 66 | 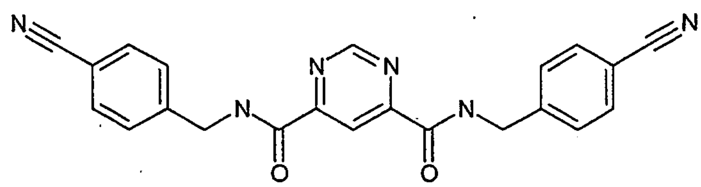 | 397,21 ES+ |
| 67 | 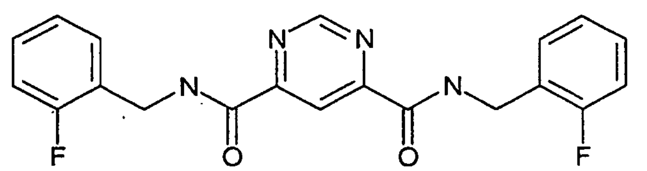 | 383,27 ES+ |
| Pharmakologische Beispiele<br>Bestimmung der enzymatischen Aktivität der katalytischen Domäne der humanen Kollagenase -3 (MMP-13). | | |

Dieses Protein wird als inaktives Pro-Enzym von der Fa. INVITEK, Berlin, erhalten (Katalog Nr. 30100 803).
Aktivierung des Proenzyms:
2 Volumenanteile Proenzym werden mit 1 Volumenanteil APMA-Lösung bei 37 ˚C für 1,5 Stunden inkubiert. Die APMA-Lösung wird aus einer 10 mmol/L p-Aminophenyl-Mercuric Acetate Lösung in 0,1 mmol/L NaOH durch Verdünnen mit

3 Volumenteile Tris/HCl Puffer pH7,5 (siehe unten) hergestellt. Der pH-Wert wird durch Zugabe von 1 mmol/L HCl zwischen 7;0 und 7,5 eingestellt. Nach der Aktivierung des Enzyms wird dieses mit dem Tris/HCl Puffer auf eine Konzentration von 1,67 μg/mL verdünnt.

Zur Messung der Enzymaktivität werden 10 μL Enzymlösung mit 10 μL einer 3%igen (v/v) gepufferten Dimethylsulfoxid-Lösung (Reaktion 1) für.15 Minuten inkubiert. Zur Messung der Enzyminhibitoraktivität werden 10 μL Enzymlösung mit 10 μL einer 3%igen (v/v) gepufferten Dimethylsulfoxid-Lösung, die den Enzyminhibitor enthält, inkubiert (Reaktion 2).

Sowohl bei Reaktion 1 als auch bei Reaktion 2 wird nach Zugabe von 10 μL einer 3%igen (v/v) wässrigen Dimethylsulfoxid-Lösung, die 0,75 mmol/L des Substrates enthält, die Enzymreaktion fluoreszenzspektroskopisch verfolgt (328 nm (Extinktion/ 393 nm(Emission)).

Die Enzymaktivität wird dargestellt als Extinktionszunahme/Minute.
Die Inhibitorwirkung wird als prozentuale Hemmung nach folgender Formel berechnet:

$$\% \text{ Hemmung} = 100 - [(\text{Extinktionszunahme/Minute in Reaktion 2}) / (\text{Extinktionszunahme/Minute in Reaktion 1}) \times 100].$$

Der $IC_{50}$, d.h. die für eine 50 %ige Hemmung der Enzymaktivität erforderliche Inhibitorkonzentration wird grafisch durch Auftragen der prozentualen Hemmungen bei verschiedenen Inhibitorkonzentrationen ermittelt.
Die Pufferlösung enthält 0,05% Brij (Sigma, Deisenhofen, Deutschland) sowie 0,1 mol/L Tris/HCl, 0,1 mol/L NaCl, 0,01 mol/L $CaCl_2$ (pH=7,5).
Die Enzymlösung enthält 1,67 μg/mL der Enzymdomäne.
Die Substratlösung enthält 0,75 mmol/L des fluorogenen Substrates (7-Methoxycoumarin-4-yl)acetyl-Pro-Leu-Gly-Leu-3-(2',4'-dinitrophenyl)-L-2,3-diaminopropionyl-Ala-Arg-NHz (Bachem, Heidelberg, Deutschland).

Die nachfolgende Tabelle 4 zeigt die Ergebnisse.

Tabelle 4

| Beispiel | IC50 MMP13 (nM) | Beispiel | IC50 MMP13 (nM) | Beispiel | IC50 MMP13 (nM) |
|---|---|---|---|---|---|
| 1 | 400 | 10 | 300 | 19 | 57 |
| 2 | 23 | 11 | 300 | 20 | 14 |
| 3 | 5600 | 12 | 260 | 21 | 10 |
| 4 | 3400 | 13 | 210 | 22 | 9 |
| 5 | 2000 | 14 | 200 | 23 | 8 |
| 6 | 700 | 15 | 190 | | |
| 7 | 620 | 16 | 105 | | |
| 8 | 400 | 17 | 80 | | |
| 9 | 320 | 18 | 72 | | |

Vergleichsbeispiel

Die Verbindung Pyrimidin-4,6-dicarbonsäurediethylamid wurde hergestellt wie in EP 0418797 beschrieben. Bei der Bestimmung des IC50-Wertes für die Inhibition der humanen Kollagenase -3 (MMP-13) wie im obengenannten Beispiel beschrieben ergibt sich ein Wert von 90 000 nM. Damit ist diese Verbindung praktisch unwirksam bei der Inhibition von MMP 13.

Bestimmung der enzymatischen Aktivität der katalytischen Domäne der humanen Neutrophilen-Kollagenase (MMP-8) und des humanen Stromelysins (MMP-3).
Die Enzyme humane Neutrophilen-Kollagenase und humanes Stromelysin wurden wie in Weithmann et al Inflamm Res, 46 (1997), Seiten 246-252, beschrieben, als aktive katalytische Domänen hergestellt, durchgeführt. Die Messung der Enzymaktivität sowie die Bestimmung der inhibitorischen Wirkung von Hemmstoffen auf die Enzymaktivität erfolgte ebenfalls wie dort beschrieben.
Die Verbindungen gemäß der obengenannten Beispiele 1 bis 23 zeigten bei der Bestimmung der humanen Neutrophilen-Kollagenase und des humanen Stromelysin jeweils IC50-Werte von mehr als 100000 nM. Damit sind diese Verbindungen

praktisch unwirksam bei der Inhibition von MMP 3 und 8.

**Patentansprüche**

**1.** Verbindung der Formel I,

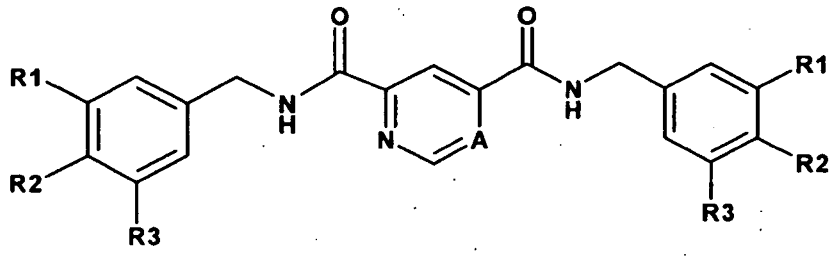

(I)

und/oder alle stereoisomere Formen der Verbindung der Formel I und/oder Gemische diese Formen in jedem Verhältnis, und/oder ein physiologisch verträgliches Salz der Verbindung der Formel I, wobei
A für ein Stickstoffatom steht,
R1 und R3 gleich oder verschieden sind und unabhängig voneinander für

1. Wasserstoffatom,
2. -($C_1$-$C_4$)-Alkyl, worin Alkyl ein-, zwei- oder dreifach substituiert ist durch Halogen, oder
3. -O-($C_1$-$C_4$)-Alkyl, worin Alkyl ein-, zwei- oder dreifach substituiert ist durch Halogen, stehen, und

R2 für

1. Wasserstoffatom,
2. Halogen,
3. -O-($C_1$-$C_4$)-Alkyl oder
4. -($C_1$-$C_4$)-Alkyl steht,

mit Ausnahme des Falles, dass

a) die Reste R1, R2 und R3 alle gleichzeitig Wasserstoffatom sind oder
b) alle Reste R1, R2 und R3 unabhängig voneinander nur Wasserstoffatom, Halogen, -($C_1$-$C_4$)-Alkyl oder -($C_1$-$C_4$)-Alkoxy bedeuten, oder

R1 und R2 oder R2 und R3 zusammen mit den Kohlenstoffatomen an die sie jeweils gebunden sind einen Dihydrofuran-Ring bilden und
der andere Rest R1 oder R3 für

1. Wasserstoffatom,
2. Halogen,
3. -($C_1$-$C_4$)-Alkyl, worin Alkyl unsubstituiert oder ein-, zwei- oder dreifach substituiert ist durch Halogen, oder
4. -O-($C_1$-$C_4$)-Alkyl, worin Alkyl ein-, zwei- oder dreifach substituiert ist durch Halogen, steht.

**2.** Verbindung der Formel I gemäß Anspruch 1, wobei
A für ein Stickstoffatom steht,
R1 und R3 gleich oder verschieden sind und unabhängig voneinander für

1. Wasserstoffatom oder
2. Trifluormethyl, stehen,

worin R1, R2 und R3 nicht gleichzeitig Wasserstoffatom sind, und
R2 für

1. Wasserstoffatom,
2. Chlor,
3. Fluor,
4. Methoxyl oder
5. Methyl steht, oder

R1 und R2 oder R2 und R3 zusammen mit den Kohlenstoffatomen an die sie jeweils gebunden sind einen Dihydrofuran-Ring bilden und
der andere Rest R1 oder R3 für

1. Wasserstoffatom,
2. Chlor,
3. Fluor,
4. Trifluormethyl oder
5. Methyl steht.

**3.** Verfahren zur Herstellung der Verbindung der Formel I gemäß der Ansprüche 1 und 2, **dadurch gekennzeichnet, dass** man eine Verbindung der Formel II

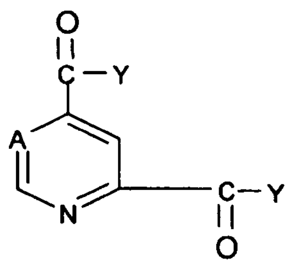

( II )

a) mit einer Verbindung der Formel III

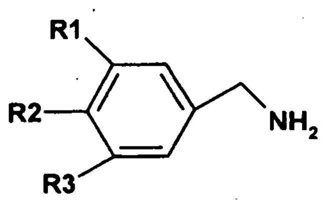

III

umsetzt, wobei R1, R2 und R3 die in Formel I gemäß Anspruch 1 angegebenen Bedeutungen haben und Y Halogen, Hydroxy oder $C_1$-$C_4$-Alkoxy ist oder zusammen mit der Carbonylgruppe einen Aktivester oder ein gemischtes Anhydrid bildet, wobei eine Verbindung der Formel 1 gebildet wird und die Reaktionsprodukte gegebenenfalls in ihre physiologisch verträglichen Salze überführt, oder
b) eine Verbindung der Formel II mit einer Verbindung der Formel III zu einer Verbindung der Formel IV.

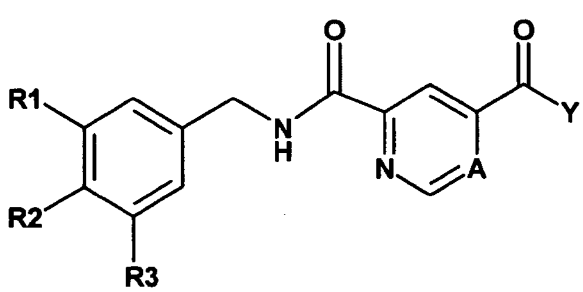

(IV)

umsetzt, wobei R1, R2 und R3 die in Formel I gemäß Anspruch 1 angegebenen Bedeutungen haben und Y Halogen, Hydroxy oder $C_1$-$C_4$-Alkoxy ist oder zusammen mit der Carbonylgruppe einen Aktivester oder ein gemischtes Anhydrid bildet, und anschließend die erhaltene Verbindung der Formel IV gegebenenfalls reinigt und anschließend mit einer Verbindung der Formel III in eine Verbindung der Formel I überführt.

**4.** Arzneimittel, **gekennzeichnet durch** einen wirksamen Gehalt von mindestens einer Verbindung der Formel I gemäß der Ansprüche 1 und 2 zusammen mit einem pharmazeutisch geeigneten und physiologisch verträglichen Trägerstoff, Zusatzstoff und/oder anderen Wirk- und Hilfsstoffen.

**5.** Verwendung der Verbindung der Formel I

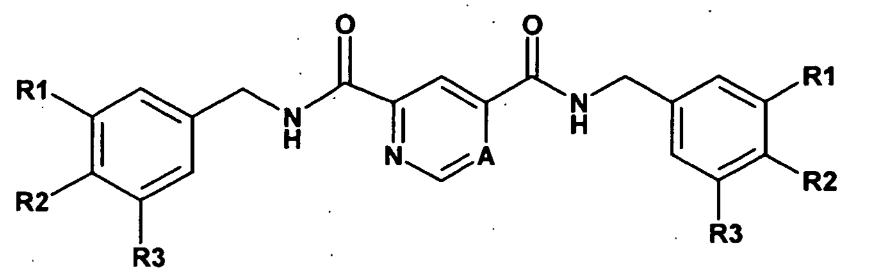

(I)

und/oder alle stereoisomere Formen der Verbindung der Formel I und/oder Gemische diese Formen in jedem Verhältnis, und/oder ein physiologisch verträgliches Salz der Verbindung der Formel I
zur Herstellung eines Arzneimittels zur Prophylaxe und Therapie von Spondylosen, Knorpelschwund nach Gelenktrauma oder längerer Gelenksruhigstellung nach Meniskus- oder Patellaverletzungen oder Bänderrissen, Kollagenosen, Arthropathien und Myalgien, wobei
A für ein Kohlenstoffatom oder Stickstoffatom steht,
R1 und R3 gleich oder verschieden sind und unabhängig voneinander für

1. Wasserstoffatom,
2. Halogen,
3. -($C_1$-$C_4$)-Alkyl, worin Alkyl unsubstituiert oder ein-, zwei- oder dreifach substituiert ist durch Halogen,
4. -O-($C_1$-$C_4$)-Alkyl, worin Alkyl unsubstituiert oder ein-, zwei- oder dreifach substituiert ist durch Halogen,
5. -C(O)-O-R4, worin R4 Wasserstoffatom oder -($C_1$-$C_4$)-Alkyl bedeutet,
6. -CN,
7. -N(R5)(R6), worin R5 und R6 gleich oder verschieden sind und unabhängig voneinander

    1. Wasserstoffatom,
    2. -($C_1$-$C_4$)-Alkyl,
    3. -C(O)-($C_1$-$C_4$)-Alkyl oder
    4. -$SO_2$-($C_1$-$C_4$)-Alkyl bedeuten,

8. -OH,
9. -S-($C_1$-$C_4$)-Alkyl,
10. -S(O)-($C_1$-$C_4$)-Alkyl,
11. -$S(O)_2$-R7, worin R7 -($C_1$-$C_4$)-Alkyl, -OH oder -$NH_2$ bedeutet, stehen,

R2 für

1. Wasserstoffatom,
2. Halogen,
3. -O-($C_1$-$C_4$)-Alkyl,
4. -($C_1$-$C_4$)-Alkyl,
5. -C(O)-O-R4, worin R4 Wasserstoffatom oder -($C_1$-$C_4$)-Alkyl bedeutet,
6. -CN,
7. -N(R5)(R6), worin R5 und R6 gleich oder verschieden sind und unabhängig voneinander

    1. Wasserstoffatom,

2. $-(C_1-C_4)$-Alkyl,
3. $-C(O)-(C_1-C_4)$-Alkyl oder
4. $-SO_2-(C_1-C_4)$-Alkyl bedeuten,

8. -OH,
9. $-S-(C_1-C_4)$-Alkyl,
10. $-S(O)-(C_1-C_4)$-Alkyl,
11. $-S(O)_2$-R7, worin R7 $-(C_1-C_4)$-Alkyl, -OH oder $-NH_2$ bedeutet, steht oder

R1 und R2 oder R2 und R3 zusammen mit den Kohlenstoffatomen an die sie jeweils gebunden sind einen 5- oder 6-gliedrigen Ring bilden, der aromatisch oder gesättigt ist und Null, ein oder zwei Heteroatome aus der Reihe Sauerstoff, Stickstoff oder Schwefel enthält und
der andere Rest R1 oder R3 die obengenannte Bedeutung von 1. bis 11. hat.

**6.** Verwendung gemäß Anspruch 5, **dadurch gekennzeichnet, dass** eine Verbindung der Formel I eingesetzt wird, wobei
A für ein Kohlenstoffatom oder Stickstoffatom steht,
R1 und R3 gleich oder verschieden sind und unabhängig voneinander für

1. Wasserstoffatom,
2. Halogen,
3. $-(C_1-C_4)$-Alkyl, worin Alkyl unsubstituiert oder ein-, zwei- oder dreifach substituiert ist durch Halogen,
4. $-O-(C_1-C_4)$-Alkyl, worin Alkyl unsubstituiert oder ein-, zwei- oder dreifach substituiert ist durch Halogen, stehen,

R2 für

1. Wasserstoffatom,
2. Halogen,
3. $-O-(C_1-C_4)$-Alkyl oder
4. $-(C_1-C_4)$-Alkyl steht, oder

R1 und R2 oder R2 und R3 zusammen mit den Kohlenstoffatomen an die sie jeweils gebunden sind einen 5- oder 6-gliedrigen Ring bilden, der aromatisch oder gesättigt ist und Null, ein oder zwei Heteroatome aus der Reihe Sauerstoff, Stickstoff oder Schwefel enthält und
der andere Rest R1 oder R3 die obengenannte Bedeutung von 1. bis 4. hat.

**7.** Verwendung gemäß der Ansprüche 5 und 6, **dadurch gekennzeichnet, dass** eine Verbindung der Formel I eingesetzt wird, wobei
A für ein Kohlenstoffatom oder Stickstoffatom steht,
R1 und R3 gleich oder verschieden sind und unabhängig voneinander für

1. Wasserstoffatom,
2. Chlor,
3. Fluor,
4. Trifluormethyl,
5. Methoxyl,
6. Methyl,
7. -C(O)-OH,
8. $-C(O)-O-CH_3$,
9. -CN,
10. $-NH_2$,
11. $-NH-C(O)-CH_3$,
12. $-NH-SO_2-CH_3$,
13. $-N-(CH_3)_2$,
14. $-SO_2-NH_2$,
15. -OH,
16. $-O-CH_2-(CHF_2)$,

17. $-S-CH_3$,
18. $-S(O)-CH_3$,
19. $-S(O)_2-CH_3$ oder
20. Brom, stehen,

R2 für

1. Wasserstoffatom,
2. Chlor,
3. Fluor,
4. Methoxyl,
5. Methyl,
6. Brom,
7. -C(O)-OH,
8. $-C(O)-O-CH_3$, .
9. -CN,
10. $-NH_2$,
11. $-NH-C(O)-CH_3$,
12. $-NH-SO_2-CH_3$,
13. $-N-(CH_3)_2$,
14. $-SO_2-NH_2$,
15. -OH,
16. $-O-CH_2-(CHF_2)$,
17. $-S-CH_3$,
18. $-S(O)-CH_3$ oder
19. $-S(O)_2-CH_3$, steht, oder

R1und R2 oder R2 und R3 zusammen mit den Kohlenstoffatomen an die sie jeweils gebunden sind einen Dioxolan-, Dihydrofuran- oder Furan-Ring bilden und der andere Rest R1 oder R3 die obengenannte Bedeutung von 1. bis 20. hat.

**8.** Verwendung gemäß einem oder mehreren der Ansprüche 5 bis 7, **dadurch gekennzeichnet, dass** eine Verbindung der Formel I eingesetzt wird, wobei
A für ein Kohlenstoffatom oder Stickstoffatom steht,
R1 und R3 gleich oder verschieden sind und unabhängig voneinander für

1. Wasserstoffatom,
2. Chlor,
3. Fluor,
4. Trifluormethyl,
5. Methoxyl, oder
6. Methyl, stehen,

R2 für

1. Wasserstoffatom,
2. Chlor,
3. Fluor,
4. Methoxyl oder
5. Methyl steht, oder

R1und R2 oder R2 und R3 zusammen mit den Kohlenstoffatomen an die sie jeweils gebunden sind einen Dioxolan-, Dihydrofuran- oder Furan-Ring bilden.

## Claims

**1.** A compound of the formula I,

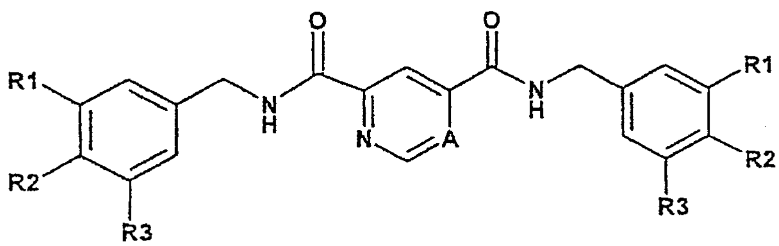

(I)

and/or all the stereoisomeric forms of the compound of the formula I and/or mixtures of these forms in any ratio, and/or a physiologically tolerated salt of the compound of the formula I, wherein
A is a nitrogen atom,
R1 and R3 are identical or different and are, independently of each other

1. hydrogen atom,
2. -($C_1$-$C_4$)-alkyl, in which alkyl is substituted once, twice or three times by halogen, or
3. -O-($C_1$-$C_4$)-alkyl, in which alkyl is substituted once, twice or three times by halogen, and

R2 is

1. hydrogen atom,
2. halogen,
3. -O-($C_1$-$C_4$)-alkyl, or
4. -($C_1$-$C_4$)-alkyl,

with the exception of the case where

a) the radicals R1, R2 and R3 are all simultaneously hydrogen atom, or
b) all the radicals from R1, R2 and R3 are, independently of each other, only hydrogen atom, halogen, -($C_1$-$C_4$)-alkyl or -($C_1$-$C_4$)-alkoxy, or

R1 and R2 or R2 and R3 form, together with the carbon atoms to which they are in each case bonded, a dihydrofuran ring, and
the other radical R1 or R3 is

1. hydrogen atom,
2. halogen,
3. -($C_1$-$C_4$)-alkyl, in which alkyl is unsubstituted or substituted once, twice or three times by halogen, or
4. -O-($C_1$-$C_4$)-alkyl, in which alkyl is substituted once, twice or three times by halogen.

**2.** A compound of the formula I as claimed in claim 1, wherein
A is a nitrogen atom,
R1 and R3 are identical or different and are, independently of each other,

1. hydrogen atom, or
2. trifluoromethyl,

in which R1, R2 and R3 are not simultaneously hydrogen atom, and
R2 is

1. hydrogen atom,
2. chlorine,
3. fluorine,
4. methoxy or
5. methyl, or

R1 and R2 or R2 and R3 form, together with the carbon atoms to which they are in each case bonded, a dihydrofuran

ring and
the other radical R1 or R3 is

1. hydrogen atom,
2. chlorine,
3. fluorine,
4. trifluoromethyl, or
5. methyl.

**3.** A process for preparing the compound of the formula I as claimed in claims 1 and 2, which comprises reacting a compound of the formula II

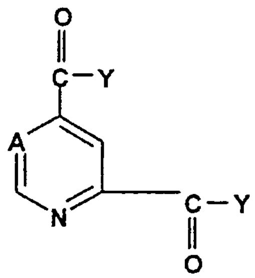

( II )

a) with a compound of the formula III

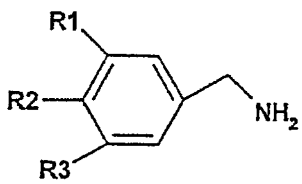

III

where R1, R2 and R3 have the meanings given in formula I as claimed in claim 1 and Y is halogen, hydroxyl or $C_1$-$C_4$-alkoxy or, together with the carbonyl group, forms an active ester or a mixed anhydride, with a compound of the formula I being formed and the reaction products being converted, where appropriate, into their physiologically tolerated salts, or
b) reacting a compound of the formula II with a compound of the formula III to give a compound of the formula IV

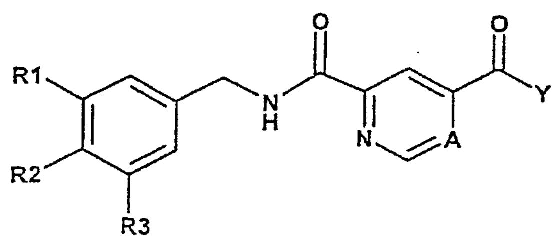

(IV)

where R1, R2 and R3 have the meanings given in formula I as claimed in claim 1 and Y is halogen, hydroxy or $C_1$-$C_4$-alkoxy or, together with the carbonyl group, forms an active ester or a mixed anhydride, and then purifying the compound of the formula IV obtained,
where appropriate, and then converting it, with a compound of the formula III, into a compound of the formula I.

**4.** A pharmaceutical which comprises an effective content of at least one compound of the formula I as claimed in claims 1 and 2 together with a pharmaceutically suitable and physiologically tolerated carrier substance, additive and/or other active compounds and auxiliary substances.

**5.** The use of the compound of the formula I

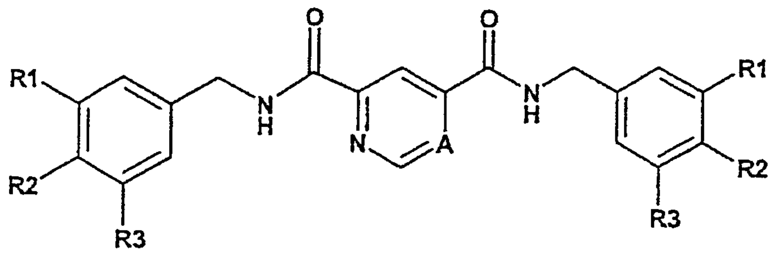

(I)

and/or all the stereoisomeric forms of the compound of the formula I and/or mixtures of these forms in any ratio, and/or a physiologically tolerated salt of the compound of the formula I
for producing a pharmaceutical for the prophylaxis and therapy of spondyloses, chondrolysis following joint trauma or a relatively long period of joint immobilization following injuries to the meniscus or patella or tearing of a ligament, collagenoses, arthropathies and myalgias,
wherein
A is a carbon atom or nitrogen atom,
R1 and R3 are identical or different and are, independently of each other,

1. hydrogen atom,
2. halogen,
3. -($C_1$-$C_4$)-alkyl, in which alkyl is unsubstituted or substituted once, twice or three times by halogen,
4. -O-($C_1$-$C_4$)-alkyl, in which alkyl is unsubstituted or substituted once, twice or three times by halogen,
5. -C(O)-O-R4, in which R4 is hydrogen atom or -($C_1$-$C_4$)-alkyl,
6. -CN,
7. -N(R5)(R6), in which R5 and R6 are identical or different and are, independently of each other,

    1. hydrogen atom,
    2. -($C_1$-$C_4$)-alkyl,
    3. -C(O)-($C_1$-$C_4$)-alkyl or
    4. -$SO_2$-($C_1$-$C_4$)-alkyl,

8. -OH,
9. -S-($C_1$-$C_4$)-alkyl,
10. -S(O)-($C_1$-$C_4$)-alkyl,
11. -$S(O)_2$-R7, in which R7 is -($C_1$-$C_4$)-alkyl, -OH or -$NH_2$,

R2 is

1. hydrogen atom,
2. halogen,
3. -O-($C_1$-$C_4$)-alkyl,
4. -($C_1$-$C_4$)-alkyl,
5. -C(O)-O-R4, in which R4 is hydrogen atom or -($C_1$-$C_4$)-alkyl,
6. -CN,
7. -N(R5)(R6), in which R5 and R6 are identical or different and are, independently of each other,

    1. hydrogen atom,
    2. -($C_1$-$C_4$)-alkyl,
    3. -C(O)-($C_1$-$C_4$)-alkyl or
    4. -$SO_2$-($C_1$-$C_4$)-alkyl,

8. -OH,
9. -S-($C_1$-$C_4$)-alkyl,
10. -S(O)-($C_1$-$C_4$)-alkyl,
11. -$S(O)_2$-R7, in which R7 is -($C_1$-$C_4$)-alkyl, -OH or -$NH_2$, or R1 and R2 or R2 and R3 form, together with the

carbon atoms to which they are in each case bonded, a 5- or 6-membered ring which is aromatic or saturated and contains zero, one or two heteroatoms from the series oxygen, nitrogen or sulfur, and the other radical R1 or R3 has the abovementioned meaning of 1. to 11.

**6.** The use as claimed in claim 5, which comprises employing a compound of the formula I wherein
A is a carbon atom or nitrogen atom,
R1 and R3 are identical or different and are, independently of each other,

1. hydrogen atom,
2. halogen,
3. -($C_1$-$C_4$)-alkyl, in which alkyl is unsubstituted or substituted once, twice or three times by halogen,
4. -O-($C_1$-$C_4$)-alkyl, in which alkyl is unsubstituted or substituted once, twice or three times by halogen,

R2 is

1. hydrogen atom,
2. halogen,
3. -O-($C_1$-$C_4$)-alkyl, or
4. -($C_1$-$C_4$)-alkyl, or

R1 and R2 or R2 and R3 form, together with the carbon atoms to which they are in each case bonded, a 5- or 6-membered ring which is aromatic or saturated and contains zero, one or two heteroatoms from the series oxygen, nitrogen or sulfur, and
the other radical R1 or R3 has the abovementioned meaning of 1. to 4.

**7.** The use as claimed in claims 5 and 6, which comprises employing a compound for the formula I wherein
A is a carbon atom or nitrogen atom,
R1 and R3 are identical or different and are, independently of each other,

1. hydrogen atom,
2. chlorine,
3. fluorine,
4. trifluoromethyl,
5. methoxy,
6. methyl,
7. -C(O)-OH,
8. -C(O)-O-$CH_3$,
9. -CN,
10. -$NH_2$,
11. -NH-C(O)-$CH_3$,
12. -NH-$SO_2$-$CH_3$,
13. -N-$(CH_3)_2$,
14. -$SO_2$-$NH_2$,
15. -OH,
16. -O-$CH_2$-($CHF_2$),
17. -S-$CH_3$,
18. -S(O)-$CH_3$,
19. -$S(O)_2$-$CH_3$ or
20. bromine,

R2 is

1. hydrogen atom,
2. chlorine,
3. fluorine,
4. methoxy,
5. methyl,
6. bromine,

7. -C(O)-OH,
8. $-C(O)-O-CH_3$,
9. -CN,
10. $-NH_2$,
11. $-NH-C(O)-CH_3$,
12. $-NH-SO_2-CH_3$,
13. $-N-(CH_3)2$,
14. $-SO_2-NH_2$,
15. -OH,
16. $-O-CH_2-(CHF_2)$,
17. $-S-CH_3$,
18. $-S(O)-CH_3$, or
19. $-S(O)_2-CH_3$, or

R1 and R2 or R2 and R3 form, together with the carbon atoms to which they are in each case bonded, a dioxolane, dihydrofuran or furan ring, and
the other radical R1 or R3 has the abovementioned meaning of 1. to 20.

**8.** The use as claimed in one or more of claims 5 to 7 which comprises employing a compound of the formula I wherein
A is a carbon atom or nitrogen atom,
R1 and R3 are identical or different and are, independently of each other,

1. hydrogen atom,
2. chlorine,
3. fluorine,
4. trifluoromethyl,
5. methoxy, or
6. methyl,

R2 is

1. hydrogen atom,
2. chlorine,
3. fluorine,
4. methoxy, or
5. methyl, or

R1 and R2 or R2 and R3 form together with the carbon atoms to which they are in each case bonded, a dioxolane, dihydrofuran or furan ring.

### Revendications

**1.** Composé de formule I,

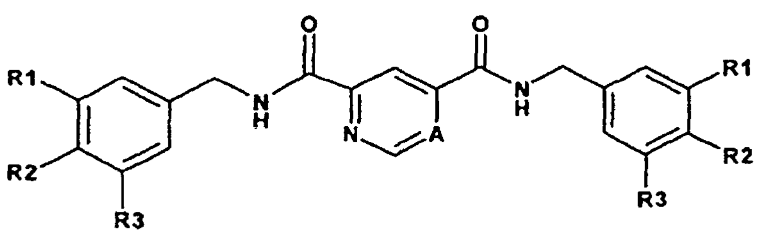

(I)

et/ou toutes les formes stéréo-isomères du composé de formule (I) et/ou les mélanges de ces formes, dans tous les rapports, et/ou un sel physiologiquement acceptable du composé de formule (I), où
A représente un atome d'azote,
R1 et R3 sont identiques ou différents et représentent, indépendamment l'un de l'autre,

1. un atome d'hydrogène
2. $-(C_1-C_4)$-alkyle, où alkyle est monosubstitué, disubstitué ou trisubstitué par halogène, ou
3. $-O-(C_1-C_4)$-alkyle, où alkyle est monosubstitué, disubstitué ou trisubstitué par halogène, et

R2 représente

1. un atome d'hydrogène
2. halogène,
3. $-O-(C_1-C_4)$-alkyle ou
4. $-(C_1-C_4)$-alkyle,

à l'exclusion du cas où

a) les radicaux R1, R2 et R3 représentent tous simultanément un atome d'hydrogène ou
b) tous les radicaux R1, R2 et R3 signifient, indépendamment l'un de l'autre, uniquement un atome d'hydrogène, halogène, $-(C_1-C_4)$-alkyle ou - $(C_1-C_4)$ -alcoxy, ou

R1 et R2 ou R2 et R3 représentent ensemble avec les atomes de carbone auxquels ils sont à chaque fois liés un cycle dihydrofuranne et l'autre radical R1 ou R3 représente

1. un atome d'hydrogène
2. halogène,
3. - $(C_1-C_4)$ -alkyle, où alkyle est non substitué ou monosubstitué, disubstitué ou trisubstitué par halogène, ou
4. $-O-(C_1-C_4)$-alkyle, où alkyle est monosubstitué, disubstitué ou trisubstitué par halogène.

**2.** Composé de formule I selon la revendication 1, où
A représente un atome d'azote,
R1 et R3 sont identiques ou différents et représentent, indépendamment l'un de l'autre,

1. un atome d'hydrogène ou
2. trifluorométhyle,

où R1, R2 et R3 ne représentent pas simultanément un atome d'hydrogène, et

R2 représente

1. un atome d'hydrogène
2. chlore,
3. fluor,
4. méthoxyle ou
5. méthyle, ou

R1 et R2 ou R2 et R3 représentent ensemble avec les atomes de carbone auxquels ils sont à chaque fois liés un cycle dihydrofuranne et l'autre radical R1 ou R3 représente

1. un atome d'hydrogène
2. chlore,
3. fluor,
4. trifluorométhyle, ou
5. méthyle.

**3.** Procédé pour la préparation du composé de formule I selon les revendications 1 et 2, **caractérisé en ce qu'**on transforme un composé de formule II

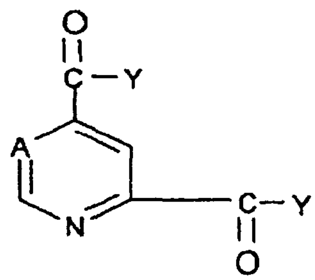

( II )

a) avec un composé de formule III

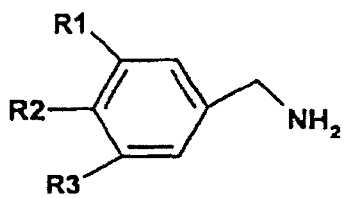

III

où R1, R2, R3 ont les significations indiquées selon la revendication 1 et Y représente halogène, hydroxy ou $C_1$-$C_4$-alcoxy ou forme, ensemble avec le groupe carbonyle, un ester actif ou un anhydride mixte, en formant un composé de formule I et on transforme le cas échéant les produits de réaction en leurs sels physiologiquement acceptables, ou

b) on transforme un composé de formule II avec un composé de formule III en un composé de formule IV

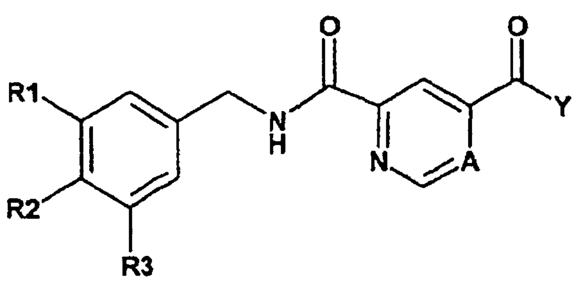

(IV)

où R1, R2, R3 ont les significations indiquées dans la formule I selon la revendication 1 et Y représente halogène, hydroxy ou $C_1$-$C_4$-alcoxy ou forme, ensemble avec le groupe carbonyle, un ester actif ou un anhydride mixte, puis on purifie le cas échéant le composé de formule IV obtenu et on le transforme ensuite avec un composé de formule III en un composé de formule I.

4. Médicament, **caractérisé par** une teneur active en au moins un composé de formule I selon les revendications 1 et 2 avec un support, un additif pharmaceutiquement approprié et physiologiquement compatible et/ou d'autres substances actives et adjuvants.

5. Utilisation du composé de formule 1

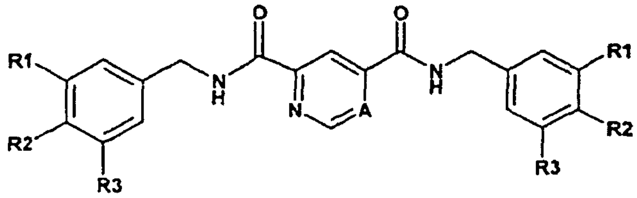

( I )

et/ou de toutes les formes stéréo-isomères du composé de formule I et/ou des mélanges de ces formes, dans tous les rapports, et/ou d'un sel physiologiquement acceptable du composé de formule I
pour la préparation d'un médicament destiné à la prophylaxie et la thérapie de spondyloses, de blessures au cartilage après un traumatisme articulaire ou une immobilisation prolongée des articulations après des blessures au ménisque ou à la rotule ou des déchirures des ligaments, des collagénoses, des arthropathies et des myalgies, où
A représente un atome de carbone ou d'azote,
R1 et R3 sont identiques ou différents et représentent, indépendamment l'un de l'autre,

1. un atome d'hydrogène
2. halogène,
3. -($C_1$-$C_4$)-alkyle, où alkyle est non substitué ou monosubstitué, disubstitué ou trisubstitué par halogène,
4. -O-($C_1$-$C_4$)-alkyle, où alkyle est non substitué ou monosubstitué, disubstitué ou trisubstitué par halogène, ou
5. -C(O)-O-R4, où R4 signifie un atome d'hydrogène ou - ($C_1$-$C_4$)-alkyle,
6. -CN
7. -N(R5)(R6), où R5 et R6 sont identiques ou différents et signifient, indépendamment l'un de l'autre,
    1. un atome d'hydrogène
    2. - ($C_1$-$C_4$) -alkyle,
    3. -C (O) - ($C_1$-$C_4$) -alkyle ou
    4. -$SO_2$-($C_1$-$C_4$)-alkyle,
8. -OH
9. -S- ($C_1$-$C_4$) -alkyle,
10. -$S(O)_2$-($C_1$-$C_4$)-alkyle,
11. -$S(O)_2$-R7, où R7 signifie -($C_1$-$C_4$)-alkyle, -OH ou -$NH_2$,

R2 représente

1. un atome d'hydrogène
2. halogène,
3. -O- ($C_1$-$C_4$) -alkyle,
4. -($C_1$-$C_4$)-alkyle,
5. -C(O)-O-R4, où R4 signifie un atome d'hydrogène ou -($C_1$-$C_4$)-alkyle,
6. -CN
7. -N(R5)(R6), où R5 et R6 sont identiques ou différents et signifient, indépendamment l'un de l'autre,
    1. un atome d'hydrogène
    2. -($C_1$-$C_4$)-alkyle,
    3. -C(O)-($C_1$-$C_4$)-alkyle ou
    4. -SO2-($C_1$-$C_4$)-alkyle,
8. -OH
9. -S-($C_1$-$C_4$)-alkyle,
10. -S(O)-($C_1$-$C_4$) -alkyle,
11. -$S(O)_2$-R7, où R7 signifie -($C_1$-$C_4$)-alkyle, -OH ou -$NH_2$,

ou
R1 et R2 ou R2 et R3 forment ensemble avec les atomes de carbone auxquels ils sont à chaque fois liés un cycle de 5 ou 6 chaînons, qui est aromatique ou saturé et qui contient zéro, un ou deux hétéroatomes de la série oxygène, azote et soufre, et l'autre radical R1 ou R3 ayant la signification susmentionnée de 1. à 11.

**6.** Utilisation selon la revendication 5, **caractérisée en ce qu'**on utilise un composé de formule I, où
A représente un atome de carbone ou d'azote,
R1 et R3 sont identiques ou différents et représentent, indépendamment l'un de l'autre,

1. un atome d'hydrogène
2. halogène,
3. - ($C_1$-$C_4$) -alkyle, où alkyle est non substitué ou monosubstitué, disubstitué ou trisubstitué par halogène,

4. -O-($C_1$-$C_4$)-alkyle, où alkyle est non substitué ou monosubstitué, disubstitué ou trisubstitué par halogène, ou

R2 représente

1. un atome d'hydrogène,
2. halogène,
3. -O- ($C_1$-$C_4$) -alkyle ou
4. -($C_1$-$C_4$)-alkyle, ou

R1 et R2 ou R2 et R3 forment ensemble avec les atomes de carbone auxquels ils sont à chaque fois liés un cycle de 5 ou 6 chaînons, qui est aromatique ou saturé et qui contient zéro, un ou deux hétéroatomes de la série oxygène, azote et soufre, et l'autre radical R1 ou R3 ayant la signification susmentionnée de 1. à 4.

**7.** Utilisation selon les revendications 5 et 6, **caractérisée en ce qu'**on utilise un composé de formule I, où A représente un atome de carbone ou d'azote,
R1 et R3 sont identiques ou différents et représentent, indépendamment l'un de l'autre,

1. un atome d'hydrogène,
2. chlore,
3. fluor,
4. trifluorométhyle,
5. méthoxyle,
6. méthyle,
7. -C(O)-OH,
8. -C(O)-O-$CH_3$,
9. -CN
10. -$NH_2$,
11. -NH-C(O)-$CH_3$,
12. -NH-$SO_2$-$CH_3$,
13. -N-$(CH_3)_2$,
14. -$SO_2$-$NH_2$,
15. -OH,
16. -O-$CH_2$-($CHF_2$) ,
17. -S-$CH_3$,
18. -S(O)-$CH_3$,
19. -$S(O)_2$-$CH_3$ ou
20. brome,

R2 représente

1. un atome d'hydrogène
2. chlore,
3. fluor,
4. méthoxyle,
5. méthyle,
6. brome,
7. -C(O)-OH,
8. -C(O)-O-$CH_3$,
9. -CN
10. -$NH_2$,
11. -NH-C(O)-$CH_3$,
12. -NH-$SO_2$-$CH_3$,
13. -N-$(CH_3)_2$,
14. -$SO_2$-$NH_2$,
15. -OH,
16. -O-$CH_2$-($CHF_2$),
17. -S-$CH_3$,
18. -S(O)-$CH_3$ ou

19. $-S(O)_2-CH_3$, ou

R1 et R2 ou R2 et R3 forment ensemble avec les atomes de carbone auxquels ils sont à chaque fois liés un cycle dioxolane, dihydrofuranne ou furanne et l'autre radical R1 ou R3 ayant la signification susmentionnée de 1. à 20.

**8.** Utilisation selon l'une ou plusieurs des revendications 5 à 7, **caractérisée en ce qu'**on utilise un composé de formule I, où
A représente un atome de carbone ou d'azote,
R1 et R3 sont identiques ou différents et représentent, indépendamment l'un de l'autre,

1. un atome d'hydrogène
2. chlore,
3. fluor,
4. trifluorométhyle,
5. méthoxyle ou
6. méthyle,

R2 représente

1. un atome d'hydrogène
2. chlore,
3. fluor,
4. méthoxyle ou
5. méthyle, ou

R1 et R2 ou R2 et R3 représentent ensemble avec les atomes de carbone auxquels ils sont à chaque fois liés un cycle dioxolane, dihydrofuranne ou furanne.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0418797 A
- EP 0463592 A
- EP 0606046 A
- WO 9428889 A
- WO 02064568 A
- WO 02064571 A


### In der Beschreibung aufgeführte Nicht-Patentliteratur


- **MASSOVA I. et al.** *The FASEB Journal,* 1998, vol. 12, 1075-1095
- Organikum, Organisch Chemisches Grundpraktikum. VEB Deutscher Verlag der Wissenschaften, 1976, 822
- **WEITHMANN et al.** *Inflamm Res,* 1997, vol. 46, 246-252